# EUROPEAN PATENT APPLICATION

(11) **EP 4 134 432 A1**
(43) Date of publication of application: **15.02.2023**
(21) Application number: 21190822.3
(22) Date of filing: 11.08.2021
(51) Int. Cl.: C12N 15/10

(54) **IMPROVED METHOD FOR SMALL RNA PURIFICATION**

(71) Applicant: Mirnagreen S.r.l., 39100 Bolzano (BZ) (IT)
(72) Inventor: VIOLA, Roberto, 39100 (BZ) Bolzano (IT)
(74) Representative: Turri, Elisa

(57) **Abstract**

The present invention refers to a method for obtaining a small RNAs (sRNAs) containing extract from fungi and/or plant or part thereof, said extract being characterized by a fraction enriched of sRNA molecules said sRNA molecules having less than 200 nucleotides or base pairs (bp), wherein said process comprises at least one step of treating said fungi or plant or part thereof with a bicarbonate solution, to obtain a liquid phase comprising or consisting of a small RNA containing extract.

## Description

### Technical field

The present invention relates to a process for obtaining a small RNAs (sRNAs) containing extract from fungi and/or plant or part thereof. Said sRNAs may comprise single stranded RNAs (ssRNAs) and/or single stranded RNAs with partial alignment and/or particularly also double stranded RNA (dsRNAs) with or without (entire) sequence complementarity. The obtained sRNA may be used to prepare or modify various products, such as to prepare second products from first products, e.g. in the nutraceutical, cosmeceutical or pharmaceutical fields.

### Background of the invention

The prior art contains various examples of methods for the isolation of small RNAs from (biological) samples, such as fungal or plant samples, and various kits for the isolation of small RNA molecules from (biological) samples, such as fungal or plant samples, are available.

Most of the known methods for isolation of small RNAs from plants or fungi have been developed for analytical scopes and are generally not suitable for efficient isolation of safe small RNA molecules from fungi and/or plants which may be used in food industry. Thus, they are usually employed with small amounts (mg or grams) of tissue and often aim at providing the full spectrum of RNA species present in the tissue. This usually involves the use of chemicals and physical strategies to prevent RNase-mediated degradation of some types or RNAs most significantly mRNAs and other ssRNAs. The strategies used to prevent RNase activity in the sample include the use of toxic solvents agents (i.e. phenol), solvents (chloroform) or chaotropic agents (TFA, PCA and guanidine HCL). All these methods are cumbersome and not amenable for scale-up or industrialization through the need for phase separation, centrifugation and/or the use (and disposal) of expensive or toxic solvents or reagents.

Convenient prior art methods generally comprise the use of a solid support to bind RNA. In WO 2017/072285, Applicant discloses extraction methods for small RNA molecules from fungi and/or plant sample requesting a step of loading the obtained solution to a solid support able to selectively bind small RNAs. Indeed, at that time, Applicant, as most of the experts in this field, considered the use of solid support, such as silica beads, essential to selectively bind and extract small RNAs from sources. This process was considered innovative for its small RNAs extraction capability over the known methods also because it does not involve the use of toxic or costly solvents.

Now, Applicant has unexpectedly found out, and repeatedly demonstrated, that the step of using solid support, such as silica beads, to bind RNA molecule is not needed or mandatory under the process conditions here disclosed. The Applicant also demonstrated that without this step the performance of the extraction process in terms of yield of small RNAs is unaffected or even improved. Moreover, without the step of using solid support to bind RNA molecules the process becomes easier to handle, shorter and less expensive.

Thus, the improved process carries with it also the advantages disclosed above for the process of WO 2017/072285.

Accordingly, it is an object of the present invention to provide such advantageous methods for purifying and obtaining, respectively, sRNAs having one or more of the said desirable features.

The present invention will be illustrated by means of non-limiting examples in reference to the following figures.

### Brief description of the drawings

**Figure 1****.** This figure shows an overview of the comparison of an exemplary method of the invention involving alcohol precipitation with correspondingly adapted alternative methods involving a solid support.
**Figure 2****.** This figure depicts results obtained when performing said comparison of methods for various types of plant and fungal samples.
**Figure 3****.** This figure shows the RNA profiles of concentrated sRNA extracts of the method comparison using the Agilent RNA 6000 Nano Kit through a Bioanalyzer instrument (Agilent Technologies).
**Figure 4****.** This figure shows the profiles of total RNA isolated by using Spectrum^{™} Plant Total RNA (Sigma-Aldrich) according to manufacturer's instructions. Profiles obtained through a Bioanalyzer instrument (Agilent Technologies) using the RNA 6000 Nano Kit (figure 4a) or the small RNA Kit (figure 4b) are shown, along with the percentages of RNA fractions of different length (20-200nt; >200nt). Small RNA represents about the 10% of the total (Fig. 4a). The distribution of the length classes of sRNAs (Fig. 4b) shows the almost total absence of RNA forms below 40nt (1% of the total).

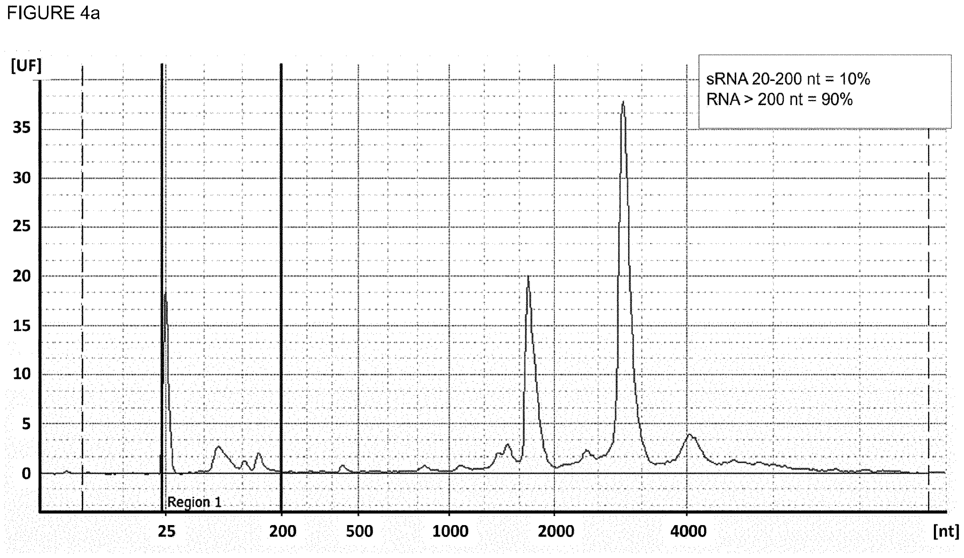

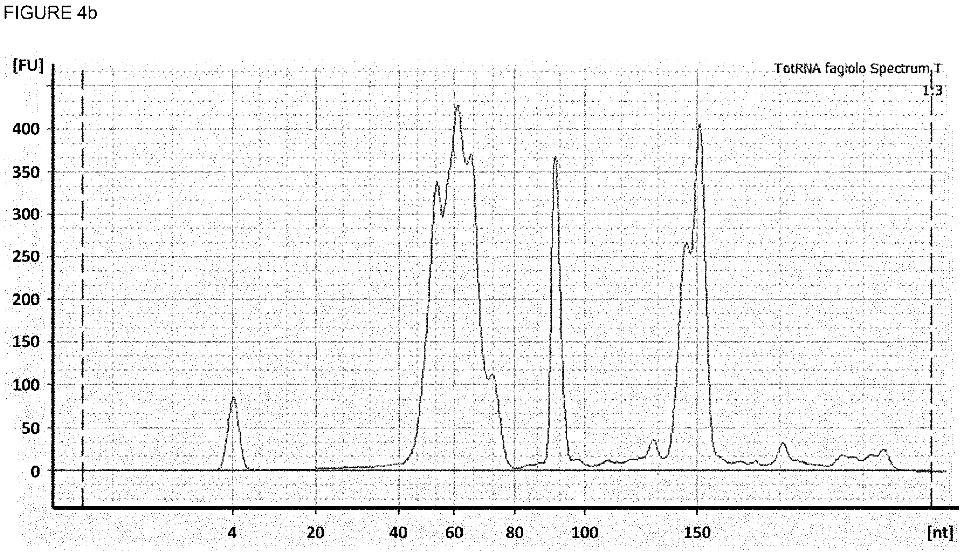

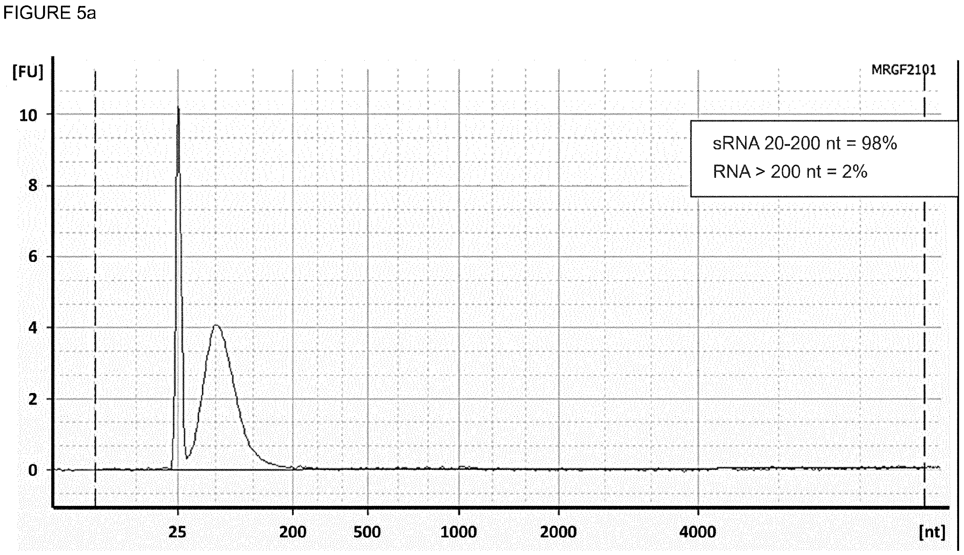

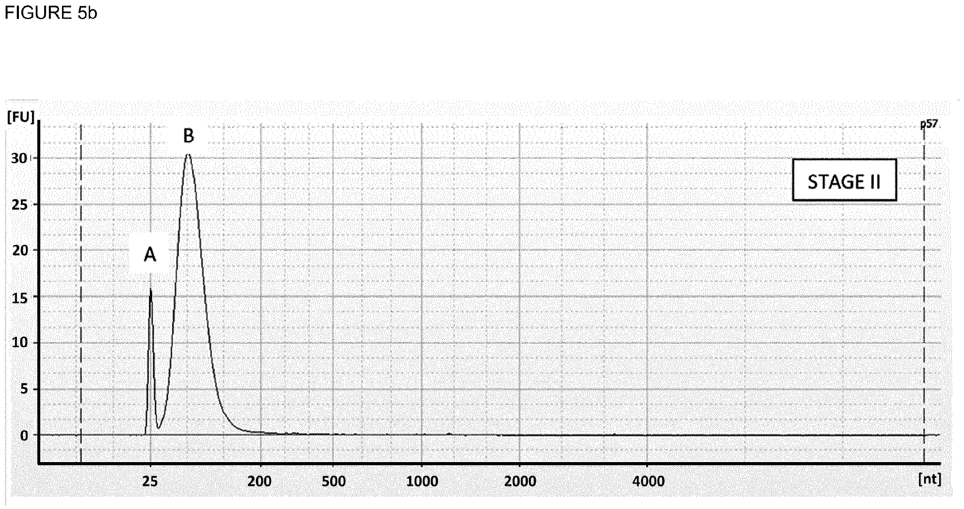

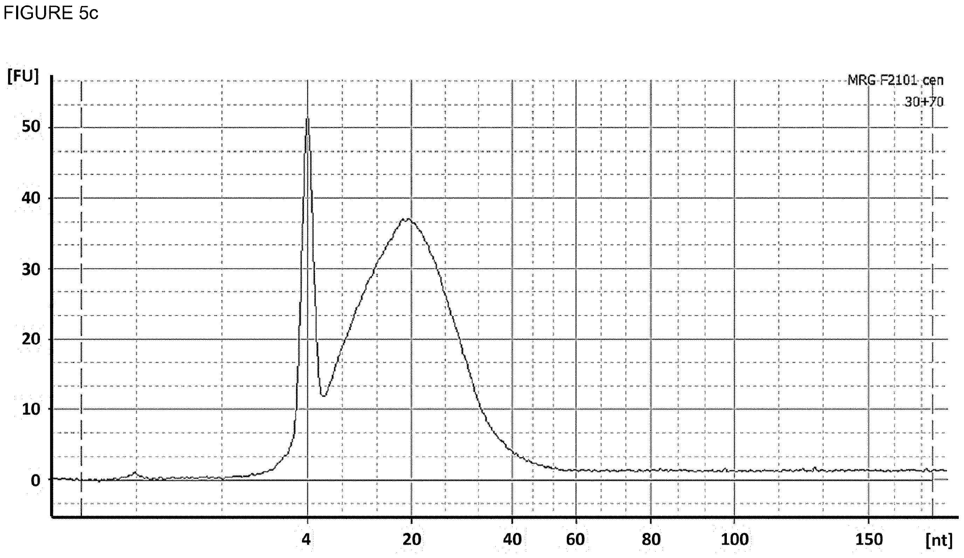

**Figure 5****.** This figure consists of two parts and provides comparative data with respect to a non-limiting exemplary method of the present invention only including bicarbonate extraction (cf. Figure 5a and 5c) and a non-limiting exemplary method that additionally includes further steps such as alcohol precipitation (cf. Figure 5b). RNA profiles obtained through a Bioanalyzer instrument (Agilent Technologies) using the RNA 6000 Nano Kit (figure 5a) or the small RNA Kit (figure 5c) are shown. As indicated, the small RNA fraction (20-200nt) represents 98% of the total RNA content in the extract. In the total RNA profile (Fig. 5a) the small RNA component represents the 98% (compared to the 10% of starting tissue (see Fig. 4)). Moreover, the distribution of the length classes of sRNAs (Fig. 5c) shows 92% of form ranging from 10 and 40 nt.

### Summary of the invention

The present invention refers to a method for obtaining a small RNAs (sRNAs) containing extract from fungi and/or plant or part thereof, said extract being characterized by a fraction enriched of sRNA molecules said sRNA molecules having less than 200 nucleotides or base pairs (bp), wherein said process comprises: a) at least one step of treating said fungi or plant or part thereof with a bicarbonate solution, to obtain a liquid phase comprising or consisting of a small RNA containing extract, providing that said process does not employ a solid support for binding small RNA molecules.

Preferably the method further comprises: b') at least one step of collecting the liquid phase of step a) and concentrating it to obtain a concentrated small RNAs containing extract; and/or b) at least one step of collecting the liquid phase of step a) or the concentrated small RNAs containing extract of step b') and adding an alcohol solution to said liquid phase of step a) or to said concentrated small RNAs containing extract of step b') to allow precipitation of the small RNAs containing extract; and optionally the following step: c) collecting the concentrated small RNAs containing extract of step b') or the precipitated sRNAs containing extract of step b).

According to a preferred embodiment, the sRNA molecules are characterized by less than 100 nucleotides, more preferably less than 30 nucleotides or bp; still more preferably said sRNAs having 19-24 nucleotides or bp, still more preferably 21-24 nucleotides or bp. Preferably the sRNA molecules represent up to 300 mg/Kg of plant or fungi processed, preferably up to 250 mg/Kg of plant or fungi processed, more preferably up to 200 mg/Kg of plant or fungi processed, still more preferably up to 100 mg/Kg of plant or fungi processed.

According to a preferred embodiment, the bicarbonate solution is selected from the group consisting of sodium bicarbonate, potassium bicarbonate, and mixtures thereof. Preferably said bicarbonate solution is used at a concentration ranging between 5 and 100 mM, preferably between 5 and 50 mM, more preferably between 5 and 30 mM, still more preferably between 5 and 10 mM or between 10 and 30 mM. More preferably said bicarbonate solution further comprises a salt, preferably NaCl, wherein said salt, preferably NaCl, is present in a concentration preferably ranging between 100 and 500 mM.

According to a preferred embodiment, said bicarbonate solution shows a pH which ranges between 7 and 9.5.

According to a preferred embodiment, the ratio between said fungi/plant and the bicarbonate solution ranges between 1:1 and 1:4.

According to a preferred embodiment, said step a) is performed at a temperature ranging between 50 to 100°C, preferably from 50 to 80°C, more preferably from 50 to 70°C, even more preferably from 60 to 70°C. More preferably step a) is performed for up to 20 hours, preferably up to 16 hours, more preferably up to 10 hours, still more preferably up to 6 or less.

According to a preferred embodiment, the liquid phase is collected by using any mean known in the art for this purpose, preferably by filtration, preferably with filters having pores with size of 10-50 µm.

According to a preferred embodiment, said alcohol is selected from isopropanol and/or ethanol and it is preferably used at a final concentration selected from: 98%, 97%, 96%, 95%, 90%, 85%, 80%, 75%, 70%, 65%, 60%, 55%, 50% v/v. Preferably the ratio liquid part: alcohol is selected from: 0.5:1, 1:1, 1,5:1, 2:1, 2,5:1, 3:1, 3,5:1, 4:1, 4,5:1.

A further aspect of the present invention refers to a product obtainable by any of the processes or methods herein disclosed.

### Detailed description of the invention

A first aspect of the present invention refers to a method for purifying small RNA molecules from a fungal and/or plant sample or part thereof, the method comprising at least one step of: a) incubating said sample with a bicarbonate solution to obtain a liquid phase containing small RNA molecules, preferably purified small RNA molecules.

Said method allows purifying small RNA molecules from samples without using any solid support for binding small RNA molecules.

Preferably the present method does not comprise the use of a solid support for binding RNA molecules after a step that comprises the addition of an alcohol solution to a liquid phase to precipitate RNA.

As used herein "method" also means process or procedure or protocol.

As used herein "purifying" also means obtaining, concentrating, isolating, or extracting.

As used herein "small RNAs (sRNAs) containing extract" means a composition comprising small RNAs deriving from a given plant or fungus or composition comprising plant or fungus material of a given plant or fungus variety wherein the amount of sRNAs of said plant variety in comparison to other RNAs of the same plant variety in said composition is higher than in naturally occurring plant material of said plant variety. In the context of the present invention the expression "small RNAs containing extract" includes the term small RNA molecules.

According to step a) said fungi/plants and/or part thereof are treated with a bicarbonate solution; in other words, said fungi/plants and/or part thereof are placed in contact or incubated with a bicarbonate solution.

Preferably said bicarbonate solution is used at a concentration ranging between 5 and 100 mM, preferably between 5 and 50 mM, more preferably between 5 and 30 mM or between 30mM and 100mM.

Optionally said bicarbonate solution may also include a salt, preferably NaCl wherein said salt, preferably NaCl, is present in a concentration preferably ranging from 100 to 500 mM, more preferably from 100 to 300 mM, still more preferably from 150 to 250 mM.

As the skilled person will readily appreciate from the data presented herein, no further method steps are obligatory after having obtained said liquid phase containing (purified) small RNA molecules.

Hence, in some preferred embodiments herein, the method of the invention consists of at least one step a), wherein step a) is optionally further defined as described elsewhere herein. According to a preferred embodiment the method further comprises at least one step b) of collecting the liquid phase of step a) and adding an alcohol solution to said liquid phase to precipitate the small RNA molecules and/or an extract comprising the small RNA molecules.

The small RNA molecules and/or the extract comprising the small RNA molecules may then be collected by any means know to the skilled in the art for the purpose. Preferably the collection of the small RNA molecules and/or the extract comprising the small RNA molecules can be done by centrifuging the liquid phase after the addition of alcohol; therefore, in this case the method of the invention further comprises a centrifuging step aimed at collecting the small RNA molecules and/or the extract comprising the small RNA molecules precipitated after the step of adding alcohol. Alternatively, the collection may be done by filtration, or any means known for this purpose.

The filtration does not use any small RNA-binding filter.

Similarly, in some embodiments herein, the method of the invention does not comprise a further step of precipitating small RNA molecules by using alcohol. Likewise, in some embodiments herein, the method of the invention does not comprise a further step of adding an alcohol solution to the liquid phase obtained in step a).

In further embodiments, additional method steps described herein may optionally be used as described herein. For example, in particular embodiments, the method further comprises the steps of: b) adding an alcohol solution to the liquid phase obtained in step a) to precipitate small RNA molecules; and c) centrifuging the precipitated small RNA molecules to obtain the small RNA molecules. As will be appreciated by the skilled reader, after said centrifugation, the small RNA molecules and/or the extract comprising the small RNA molecules are contained in the pellet meaning that the precipitated small RNA forms a sedimented portion that accumulates during centrifugation. The resulting supernatant may then be removed. Preferably the pellet containing the small RNA molecules may then optionally be resuspended.

Therefore, preferably the method of the invention comprises the steps of: a) incubating a fungal and/or plant sample with a bicarbonate solution obtaining the liquid phase of step a); b) adding an alcohol solution to the liquid phase obtained in step a) to precipitate small RNA molecules and/or the extract comprising the small RNA molecules; and i) centrifuging the precipitated small RNA molecules and/or the extract comprising the small RNA molecules to obtain a sample/extract containing purified small RNA molecules, providing that the method does not employ a solid support for binding small RNA molecules.

Preferably the method of the invention comprises:
a) At least one step of treating said fungi or plants or part thereof with a bicarbonate solution to obtain a liquid phase comprising or consisting of the small RNA containing extract;
b') At least one step of collecting the liquid phase of step a) and concentrating it to obtain a concentrated small RNAs containing extract; and
b) At least one step of collecting the concentrated small RNAs containing extract of step b') and adding an alcohol solution to said concentrated small RNAs containing extract to allow the precipitation of the small RNAs containing extract; and optionally
c) Collecting the precipitated sRNAs containing extract of step b).

Preferably the method of the invention comprises:
a) At least one step of treating said fungi or plants or part thereof with a bicarbonate solution, preferably a bicarbonate solution or a carbonate solution, more preferably a bicarbonate solution to obtain a liquid phase comprising or consisting of the small RNA containing extract;
b) At least one step of collecting the liquid phase of step b') and adding an alcohol solution to said liquid phase to allow precipitation of the small RNAs containing extract; and optionally
c) Collecting the precipitated sRNAs containing extract of step b).

Preferably the method of the invention comprises:
a) At least one step of treating said fungi or plants or part thereof with a bicarbonate solution to obtain a liquid phase comprising or consisting of the small RNA containing extract;
b') At least one step of collecting the liquid phase of step a) and concentrating it to obtain a concentrated small RNAs containing extract and
c) Collecting the concentrated small RNAs containing extract of step b').

Preferably the method of the invention comprises:
a) incubating a fungal and/or plant sample with a bicarbonate solution as defined above; obtaining the liquid phase of step a); b) adding an alcohol solution to the liquid phase obtained in step a) to precipitate small RNA molecules and/or the extract comprising the small RNA molecules; and i) centrifuging the precipitated small RNA molecules and/or the extract comprising the small RNA molecules to obtain a sample/extract containing purified small RNA molecules.

Accordingly, in certain general embodiments herein, said step b) is optionally preceded by a step b0) comprising ultrafiltrating the liquid phase obtained in step a), to obtain a sample having an increased concentration of small RNA molecules. In said filtration step, the precipitated small RNA molecules will be retained (i.e. not pass through the filter). As will be appreciated by the skilled reader, said step is advantageous e.g. if it is desired to use less alcohol for the precipitation step, as it will reduce the sample volume and hence the volume of alcohol required. The said filtration may e.g. also be employed in other cases where it is desirable to reduce the volume of the sample and/or increase the concentration of sRNA molecules in the sample. Accordingly, a particular order of steps of a method of the invention includes steps a), b0), b) and c) or i).

Preferably the pore sizes for the filtration ranges from 0.1 kDa to 10 kDa, preferably from 0.1 or to 5kDa, more preferably the for size is selected from: 0.1, 0.2, 0.3, 0.4, 0.5, 0.6, 0.7, 0.8, 0.9, 1.0, 1.1, 1.2, 1.3, 1.4,1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0.

Accordingly, in further embodiments herein, said step i) is optionally replaced by or combined with a step c') of (ultra)filtrating the precipitated small RNA molecules to obtain a sample having an increased concentration of small RNA molecules. In said filtration step, the precipitated small RNA molecules will be retained (i.e. not pass through the filter). The said filtration may e.g. be employed where it is desirable to reduce the volume of the sample and/or increase the concentration of sRNA molecules in the sample. Accordingly, a particular order of steps of a method of the invention includes steps a), b), c'), and i).

In general embodiments herein, the sample or the part of the fungus or plant is a fungal or plant sample, respectively. Herein, fungal or plant samples may be defined as samples comprising cells, preferably tissue, of at least one fungus or at least one plant.

As used herein "fungi" or "fungal" means "fungi" or "fungal samples" well known to the person skilled in the art, preferably selected from the phyla Ascomycota, Basidiomycota, Chytridiomycota, and Zygomycota as well as the Oomycota and all mitosporic fungi (as defined by Hawksworth et al. In, Ainsworth and Bisby's Dictionary of The Fungi, 8th edition, 1995, CAB International, University Press, Cambridge, UK). The fungal cell(s) can be any fungal cell, meaning any cell present within or derived from an organism belonging to the Kingdom Fungi. The methods of the invention are applicable to all fungi and fungal samples. In one embodiment of the invention, the fungus may be a mould, or more particularly a filamentous fungus. In other embodiments of the invention, the fungus may be a yeast (e.g. Saccharomyces or Pichia), or Aspergillus, Rhizopus, Neurospora. In one embodiment the fungus may be an ascomycetes fungus, i.e. a fungus belonging to the Phylum Ascomycota. More preferably said fungus is selected from: Agaricus bisporus, and fungi of the genuses Agrocybe, Amanita, Armillaria, Artomyces, Astraeus, Aureoboletus, Auricularia, Boletus, Bovista, Butyriboletus, Calbovista, Calocybe, Calvatia, Candy Cap, Cantharellula, Cantharellus, Chalciporus, Chanterelle, Chroogomphus, Clavaria, Clavariadelphus, Clavulina, Clitocybe, Clitopilus, Coprinellus, Coprinopsis, Coprinus, Corn smut, Cortinarius, Craterellus, Cyanoboletus, Cystoderma, Cystodermella, Dacryopinax, Disciotis, Entoloma, Eritadenine, Exsudoporus, Fistulina, Floccularia, Geopora, Gliophorus, Gomphidius, Gomphus, Goossensia, Grifola, Guepinia, Gymnopus, Gyromitra, Gyroporus, Handkea, Harrya, Helvella, Hemileccinum, Hericium, Hydnum, Hygrocybe, Hygrophorus, Hypomyces, Hypsizygus, Imleria, Infundibulicybe, Laccaria, Laccocephalum, Lactarius, Lactifluus, Laetiporus, Lanmaoa, Leccinellum, Leccinum, Lentinula, Lepista, Leucopholiota, Lobaria, Lycoperdon, Mackintoshia, Marasmius, Melanoleuca, Meripilus, Morchella, Mycenastrum, Penicillium, Phallus, Phylloporus, Pleurocybella, Pleurotus, Pluteus, Polyozellus, Psathyrella, Pseudohydnum, Ramaria, Ramariopsis, Rhizopogon, Rhodocybe, Russula, Saccharomyces, Sarcodon, Sarcosphaera, Sparassis, Strobilurus, Stropharia, Suillellus, Suillus, Termitomyces, Tremella, Tricholoma, Tylopilus Verpa, Volvariella, Volvopluteus, Xerocomellus, Xerocomus, Xeromphalina.

Herein, the terms fungal / fungi may relate to fungi / fungal cells of all types and at all stages of development, including specialized reproductive cells such as sexual and asexual spores. As used herein the terms fungal / fungi may relate to the fungus as such and also other life forms of the fungus, such as haustoria, conidia, mycelium, penetration peg, spore, zoospores etc.

Herein the term "fungi" or "fungal", respectively, may be replaced by the term "mushrooms" or "mushroom", respectively - and vice versa.

As used herein, "plants" and "plant samples" mean such plants or sample thereof generally well known to the person skilled in the art and are not particularly limited and preferably encompasses any member of the plant-kingdom according to the Linnaeus definition. Preferably the plants are selected from the genera Arabidopsis, preferably Arabidopsis thaliana, or Phaseolus, preferably Phaseolus vulgaris, or Nicotiana, preferably Nicotiana tabacum, or Glycine, preferably Glycine max, or Gossypium, preferably Gossypium arboreum, or Brassica, preferably Brassica napus, or Vitis, preferably Vitis vinifera, or Beta, preferably Beta vulgaris, or Triticum, preferably Triticum aestivum, or Solanum, preferably Solanum lycopersicum, Solanum tuberosum and Solanum melongena L., or Musa, preferably Musa acuminata and Musa balbisiana, or Fragaria, preferably Fragaria vesca, Fragaria viridis and Fragaria moschata, or Oryza, preferably Oryza sativa, or Hordeum, preferably Hordeum vulgare, or Olea, preferably. Olea europaea, or Malus, preferably Malus domestica, or Allium, preferably Alluim cepa, or Pisum, preferably Pisum sativum, or Mentha, or Cuminum, preferably Cuminum cyminum. The term plant also comprises lychens and algae and fruitings and any part of the plant.

Any part of the plant can be used for the purpose of the invention, preferably said part is selected from: cotyledons, leaves (fresh or dry), sprouts, root, flowers, bulb, seed and/or fruits.

In the present invention, the bicarbonate solution preferably comprises an alkali metal bicarbonate. Accordingly, the term "bicarbonate solution" may herein be replaced by "alkali metal bicarbonate solution". Said alkali metal bicarbonate herein is preferably selected from the group consisting of sodium bicarbonate (NaHCO₃), potassium bicarbonate (KHCO₃), and mixtures thereof.

In preferred embodiments herein, the bicarbonate solution comprises NaHCO₃. Accordingly, the term "bicarbonate solution" may herein be replaced by "NaHCO₃ solution". In other embodiments herein, the bicarbonate solution comprises KHCO₃. Accordingly, the term "bicarbonate solution", may herein be replaced by "KHCO₃ solution". In certain preferred embodiments herein, the bicarbonate solution i) comprises from 5 to 100 mM NaHCO₃, particularly from 10 to 50 mM NaHCO₃, especially from 20 to 40 mM NaHCO₃, and/or ii) comprises from 100 to 500 mM NaCl, particularly from 100 to 300 mM NaCl, especially from 150 to 250 mM NaCl; especially wherein the bicarbonate solution comprises from 20 to 40 mM NaHCO₃ and from 150 to 250 mM NaCl.

Accordingly, in the present invention, the bicarbonate solution preferably comprises from 5 to 100 mM bicarbonate, particularly from 10 to 50 mM bicarbonate, especially from 20 to 40 mM bicarbonate.

As used herein, the term "about" in connection with values preferably means, that a given amount is in a range around the indicated amount, which reaches from minus 10%, preferably from minus 5% of the indicated amount to plus 10%, preferably plus 5% of the indicated amount. Accordingly, in preferred embodiments herein, a term "about x" may be replaced by "x ± 10%", more preferably by "x ± 5%".

In certain embodiments herein, the bicarbonate solution additionally comprises from 100 to 500 mM NaCl, particularly from 100 to 300 mM NaCl, especially from 150 to 250 mM NaCl.

In certain specific embodiments herein, the bicarbonate solution comprises from 20 to 40 mM bicarbonate and from 150 to 250 mM NaCl.

According to a preferred embodiment, to a given (starting) sample is added a bicarbonate solution in an amount of from about 1:1 to about 1:25 (w/v) depending on the kind of tissue, e.g. if fresh, or dried, or lyophilized. Preferably the ratio bicarbonate solution sample is 1:10 or 1:3(w/v).

Generally, in the present invention, an alcohol solution comprises at least one alcohol, preferably at least one alcohol capable of promoting the precipitation or insolubilization of small RNA molecules, and more preferably at least one alcohol selected from: isopropanol, ethanol, propanol, methanol, butanol and/or any other alcohol capable of promoting the precipitation or insolubilization of small RNA molecules, preferably the alcohol is isopropanol and/or ethanol.

The amount of the alcohol generally depends on the alcohol used, but are readily known or can readily be determined by a person skilled in the art.

In a preferred embodiment the alcohol is added to the liquid phase in a ratio preferably selected from: 0.5:1, 1:1, 1,5:1, 2:1, 2,5:1, 3:1, 3,5:1, 4:1, 4,5:1 (v/v).

In certain preferred embodiments herein, particularly in any washing step of the methods of the invention, a given alcohol solution comprises isopropanol, especially from 90% to 100% isopropanol, preferably from 95% to 100% isopropanol. Accordingly, in certain embodiments herein, a given alcohol solution "added in a washing step"(which may also be referred to herein as "added in a step involving washing of a pellet containing small RNA molecules") comprises isopropanol, especially from 90% to 100% isopropanol, in particular from 95% to 100% isopropanol. In certain preferred embodiments herein, particularly in any washing step of the methods of the invention, a given alcohol solution comprises ethanol, especially from 70% to 100% ethanol, preferably 70% to 80% ethanol. Accordingly, in certain embodiments herein, a given alcohol solution "added in a washing step"(which may also be referred to herein as "added in a step involving washing of a pellet containing small RNA molecules") comprises ethanol, especially from 70% to 100% ethanol, in particular from 70% to 80% ethanol.

Preferably the alcohol solution can be about, be at least about, or be at most about 5, 10, 15, 20, 25, 30, 35, 40, 45, 50, 55, 60, 65, 70, 75, 80, 85, 90, 95, or 100% alcohol, or any range therein. In certain embodiments, an alcohol solution is added to a sample to make the final solution have a concentration of alcohol of about, about at least, or about at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90%, or any range therein.

In certain preferred embodiments herein, particularly in any precipitation step of the methods herein a given alcohol solution is added to yield a final concentration (v/v) of about 40% to about 80% alcohol, preferably of about 50% to about 70% alcohol. According to a preferred embodiment of the invention, a washing step of the precipitate is performed. For this purpose, a given alcohol solution is added to yield a final concentration (v/v) preferably of about 70% to about 80% alcohol.

As used herein, the expression "small RNA molecules" includes any miRNA molecule, siRNA molecule, preferably selected from: heterochromatic siRNA, secondary siRNA, phased siRNA, transacting siRNA, cis-NAT-siRNA, and trans-NAT-siRNA, snRNA molecules, snoRNA molecules, tRNA molecules, siRNA fragments, tRNA fragments, mRNA fragments, rRNA fragments. Preferably said small RNA molecules are characterized by less than 200 nucleotides or base pairs (bp), preferably less than 100 nucleotides, more preferably less than 30 nucleotides or bp; still more preferably said sRNAs having 19-24 nucleotides or bp, still more preferably 21- 24 nucleotides or bp.

Preferably said sRNAs are single and/or double stranded wherein the double stranded sRNAs may be fully or partially aligned or having mismatch or having 3' overhangs.

Preferably said sRNAs are characterized by the presence of a phosphate group at the 5' ends and/or by the presence of a methyl group at the 3' ends. Accordingly, in certain embodiments herein, the small RNA molecules include miRNA molecules, siRNA molecules (particularly selected from the group consisting of heterochromatic siRNA, secondary siRNA, phased siRNA, trans-acting siRNA, cis-NAT-siRNA, and trans-NAT-siRNA), snRNA molecules, snoRNA molecules, tRNA molecules, siRNA fragments, tRNA fragments, mRNA fragments, rRNA fragments, and/or IncRNA fragments. In particular, the small RNA molecules include miRNA, siRNA (particularly selected from the group consisting of heterochromatic siRNA, secondary siRNA, phased siRNA, trans-acting siRNA, cis-NAT-siRNA, and trans-NAT-siRNA), snRNA, snoRNA, and/or tRNA molecules. Especially, the small RNA molecules include miRNA and/or siRNA (particularly selected from the group consisting of heterochromatic siRNA, secondary siRNA, phased siRNA, trans-acting siRNA, cis-NAT-siRNA, and trans-NAT-siRNA) molecules.

In context with the small RNA molecules in context with the invention, it is to be noted that the methods of the invention particularly also aim at the selective enrichment in the extracts of small RNA molecules, preferably microRNA and siRNA of length between 19 and 24 nt or bp. These small RNA molecules may be single or double stranded molecules (ssRNAs and dsRNAs) and preferably are characterized by the presence of a phosphate group at the 5' ends and/or a methyl group at the 3' ends. These properties make these molecules much more resistant than other sRNAs to degradation during extraction. Further embodiments of the present invention particularly relate to sRNAs disclosed in the publication "Plant microRNAs as novel immunomodulatory reagents", Scientific Reports 2016 Cavalieri et al (incorporated herein by reference in its entirety), which sRNAs were shown to display anti-inflammatory properties.

According to a preferred embodiment the incubation step is performed at a temperature preferably ranging from 50 to 100°C, preferably from 50 to 80°C, more preferably from 50 to 70°C, even more preferably from 60 to 70°C.

Preferably the treating step may last for up to 20 hours, preferably up to 16 hours, more preferably up to 10 hours, still more preferably up to 6 or less, e.g for 2, 3, 4, 5 or 6 hours.

In the present invention the collecting step is e.g. carried out by one or more means selected from the group consisting of filtration, centrifugation and taking off by suction. In particular preferred embodiments, step a) comprises one or more steps of collecting the liquid phase by one or more means selected from the group consisting of filtration and centrifugation. Hence, step a) preferably comprises separating the liquid phase from the solid phase by filtration and/or centrifugation, preferably by filtration or centrifugation.

Accordingly, step a) preferably comprises collecting the liquid phase by filtration, particularly where the liquid phase is not retained by the filter. Accordingly, step a) preferably comprises collecting the liquid phase by centrifugation, particularly where the liquid phase is in the supernatant.

In a particular embodiment, step a) further comprises the steps of filtrating the liquid phase, centrifuging the resulting liquid phase, and discarding the resulting pellet.

In view of the above, step a) preferably comprises a step of removing the solid phase, preferably by any of the means described herein.

In certain embodiments herein, step a) of a method of the invention is characterized in that is preceded by a further step of incubating the sample in a bicarbonate solution or water, such as at room temperature or lower temperature, particularly wherein the final concentration (v/w) thereof is half the final concentration (v/w) of the bicarbonate solution used in step a).

After the collection step or at the end of any of the method according to the invention, the extract is preferably lyophilized or dried with any method known to the skilled person. Generally, herein, the said fungal and/or plant sample for use in the invention is not particularly limited.

In certain preferred embodiments, said sample comprises intact fungal and/or plant material, such as intact fungal and/or plant tissue. In certain preferred embodiments, said sample comprises chopped, preferably coarsely chopped, fungal and/or plant material, such as chopped, preferably coarsely chopped, fungal and/or plant tissue. In certain preferred embodiments, said sample comprises ground fungal and/or plant material, such as ground fungal and/or plant tissue. Accordingly, the sample used in the methods of the invention preferably comprises, especially consists of, intact tissue, chopped tissue or ground tissue.

In certain embodiments herein, step a) of a method of the invention is characterized in that the said fungal and/or plant sample is selected from the group consisting of fungal tissue, fungal cells, plant tissue, and plant cells, particularly wherein said tissue is intact, chopped or ground, especially wherein said tissue is intact or chopped, such as coarsely chopped, in particular wherein the sample consists of intact or chopped, such as coarsely chopped, plant and/or fungal tissue. For example, with a fungal and/or plant sample of low water content, such as a dry or dried sample, said tissue is preferably intact or coarsely chopped. For example, with a fresh fungal and/or plant sample, and/or a fungal and/or plant sample with high water content, it is preferred said tissue is preferably chopped, preferably coarsely chopped.

Chopped and preferably coarsely chopped plant and/or fungal samples, tissue or the like are well-known to the skilled person. As used herein, a coarsely chopped sample, tissue or the like may be a sample, tissue or the like that has been chopped by hand or by technical means resulting in a respective fragmentation. In other embodiments herein, the fungal or plant sample, tissue or the like has been ground, preferably has been ground into a fine powder, as readily appreciated by the person skilled in the art.

Accordingly, in more detail, in certain embodiments herein, the sample comprises, preferably consists of intact plant tissue. In certain embodiments herein, the sample comprises, preferably consists of intact mushroom tissue. In certain embodiments herein, the sample comprises, preferably consists of chopped plant tissue. In certain embodiments herein, the sample comprises, preferably consists of chopped mushroom tissue. In certain embodiments herein, the sample comprises, preferably consists of ground plant tissue. In certain embodiments herein, the sample comprises, preferably consists of ground mushroom tissue. In certain embodiments herein, the sample comprises, preferably consists of intact plant cells. In certain embodiments herein, the sample comprises, preferably consists of intact mushroom cells.

Generally, herein, a sample initially employed in a method of the invention may be referred to as "starting sample". Accordingly, the starting sample of a method of the invention preferably consists of plant or fungal tissue, preferably intact tissue or chopped tissue. Thus, the starting sample of a method of the invention preferably consists of plant or fungal cells.

In preferred general embodiments herein, a method of the invention additionally comprises i) selectively removing ssRNAs with RNase treatment from the small RNA molecules, and/or ii) treating the small RNA molecules with an agent which binds with high affinity to siRNA duplexes of selectively 21-25 nucleotides (nt), to enrich 21-24 bp dsRNA; and/or iii) taking up small RNA molecules in a solution, preferably in an RNase-free aqueous solution, preferably in RNase-free water. Accordingly, in certain embodiments herein, a method of the invention additionally comprises selectively removing ssRNAs with RNase treatment from the small RNA molecules. Accordingly, in certain embodiments herein, a method of the invention additionally comprises treating the small RNA molecules with an agent which binds with high affinity to siRNA duplexes of selectively 21-25 nt, to enrich 21-24 bp dsRNA. Accordingly, in certain embodiments herein, a method of the invention additionally comprises taking up small RNA molecules in a solution, preferably in an RNase-free aqueous solution, preferably in RNase-free water. In preferred general embodiments herein, a method of the invention further comprises one or more washing steps, said washing steps optionally including centrifugation, particularly wherein the one or more washing steps include i) one or more washing steps involving washing of a pellet containing small RNA molecules with an alcohol solution, and/or ii) one or more washing steps involving taking up a pellet containing small RNA molecules in solution and removing insoluble sample constituents, preferably by centrifugation; especially wherein the method further comprises an additional precipitation step of adding an alcohol solution to a liquid phase containing small RNA molecules to precipitate small RNA molecules, optionally followed by centrifugation of the precipitated small RNA molecules, in particular wherein said additional precipitation step is preceded and/or followed by a washing step according to i) above and a washing step according to ii) above.

In certain embodiments, the one or more washing steps include i) one or more washing steps involving washing of a pellet containing small RNA molecules with an alcohol solution.

In certain embodiments, the one or more washing steps include ii) one or more washing steps involving taking up a pellet containing small RNA molecules in solution and removing insoluble sample constituents, preferably by centrifugation.

Accordingly, in certain embodiments herein, a method of the invention further includes an additional precipitation step of adding an alcohol solution to a liquid phase containing small RNA molecules to precipitate small RNA molecules, optionally followed by centrifugation of the precipitated small RNA molecules.

Said additional precipitation step may be preceded and/or followed by a washing step according to i) above and a washing step according to ii) above.

As described above, methods of the invention are characterized by the proviso that the method does not employ a solid support for binding small RNA molecules.

Preferably said solid support for binding small RNA molecules can selectively bind small RNA molecules.

In preferred general embodiments herein, said solid support is a column. In preferred general embodiments herein, the said solid support is one or more beads or comprises silica, such as silica filter.

In preferred general embodiments herein, the said solid support is a mineral or polymer support, particularly wherein i) the mineral support or polymer support is a column, especially a column comprising silica or silica gel or silicon dioxide particles, and/or ii) the mineral or polymer support is a set of beads, especially of beads made of an absorptive polymer, preferably wherein the set of beads is collectable by centrifugation, filtration, or magnetic capture.

By "silica" as used herein are preferably meant SiO₂ crystals and other forms of silicon oxide, such skeletons of diatoms built up from SiO₂, amorphous silicon oxide and glass powder. Also, alkyl silica, aluminum silicate (zeolite), activated silica with -NH₂, latex particles are examples for a solid support in context with the invention.

In embodiments of the methods of the invention, there is no use of a nucleic acid (NA) binding solid support such as silica particles capable of binding the nucleic acid in the presence of a chaotropic substance.

In the present invention, a "solid support" may refer to a physical structure containing a material which contacts the solution and reversibly binds to macromolecules in the solution, particularly to RNA molecules, more particularly to small RNA molecules. The material in the solid support may include a mineral or polymer, in which case the support may be referred to as a "mineral or polymer support." Said support is preferably elected from: mineral or polymer support, magnetic silica beads, Perspex column comprising silica gel, silicon dioxide beads, and combinations thereof.

Likewise, the solid support ("for binding small RNA molecules") described in context with the invention is not a filter used in any filtration step described herein, particularly not a filter employed in context with method step a) of the invention. Accordingly, e.g. a filter employed herein to obtain a liquid phase containing small RNA molecules (i.e. allowing the liquid phase containing small RNA molecules to flow through) does not qualify as a solid support as described in context with the invention, particularly since same is not used for binding sRNA molecules. Similarly, a filter used for ultrafiltration herein does not qualify as a solid support as described in context with the invention, particularly since same is not used for binding sRNA molecules, but e.g. to concentrate a solution comprising sRNA molecules.

Accordingly, the methods of the present invention are distinguished from prior art methods which do employ a solid support for binding small RNA molecules. For reference purposes, said prior art methods involve one or more of the following features. Thus, according to particular embodiments herein, the methods of the invention are characterized in that one or more of said particular features are not part of the methods of the present invention.

In preferred general embodiments herein, a method of the invention is further characterized in that it does not involve the use of one or more agents selected from the group consisting of surfactants, particularly SDS (sodium dodecyl sulfate) and/or CTAB (cetyltrimethylammonium bromide); complexing agents, particularly EDTA (ethylenediamine tetraacetic acid); chaotropic agents, particularly selected from guanidine hydrochloride, guanidinium thiocyanate, TFA (trifluoroacetic acid), PCA (perchloric acid), and phenol; chloroform and LiCI, particularly wherein the method does not involve the use of any of SDS and EDTA, especially wherein the method does not involve the use of any of TRIzol^{®}, CTAB and LiCI.

In preferred general embodiments herein, a method of the invention is i) a method for purifying small RNA molecules from a sample, and/or ii) a method for isolating, preferably for selectively isolating, small RNA molecules from a sample. Accordingly, the method of the invention may be a method for purifying small RNA molecules from a sample. Accordingly, the method of the invention may be a method for isolating, preferably selectively isolating, small RNA molecules from a sample.

Preferably herein, a method of the invention is i) a method for increasing the ratio of small RNA molecules to total RNA, and/or ii) a method for increasing the ratio of small RNA molecules to total nucleic acids, and/or iii) a method for isolating small RNA molecules from a sample; particularly wherein the said ratio indicates a weight ratio.

In certain embodiments, the method of the invention is a method for improving the yield of obtained small RNA molecules. In certain embodiments, the method of the invention is a method for purifying the small RNA molecules. In certain embodiments, the method of the invention is a method for obtaining, preferably selectively obtaining, the small RNA molecules. In certain embodiments, the method of the invention is a method for increasing the percentage of small RNA molecules contained in the sample. In certain embodiments, the method of the invention is a method for improving the purity of obtained small RNA molecules. In certain embodiments, the method of the invention is a method for separating the small RNA molecules from other RNA molecules in the sample. In certain embodiments, the method of the invention is a method for separating the small RNA molecules from DNA molecules in the sample. In certain embodiments, the method of the invention is a method for avoiding the use of toxic reagents. In certain embodiments, the method of the invention is a method for achieving, especially for improving, the release, particularly the selective release, of small RNA molecules from the sample.

Typically, the endogenous nucleic acids found in plant or fungal cells consist of DNA and RNA. The DNA/RNA ratio varies between species and tissues but generally can be considered a ratio 1:1 as in all eukaryotes. Small RNA molecules represent less than 10% of total RNA and thus less than 5% of total nucleic acids.

Typically, small RNA molecules represent less than 10% of the total RNA. It was found that the method of the invention results in samples wherein small RNA molecules represent much more than 50%, such as even more than 90% of total nucleic acids. Further, it was found that the method of the invention results in samples wherein almost no RNA molecules having a length of more than 200 nucleotides are detectable.

In certain embodiments of a method of the invention, the purity of the small RNA molecules resulting from the complete method is increased by a factor of at least 10, particularly at least 100, especially at least 500, in particular at least 1000, such as at least 1000, at least 3000, at least 5000 as compared to the purity of the small RNA molecules in the starting sample, particularly if measured in weight percent (w/w).

In particularly preferred embodiments, the methods of the invention achieve particular levels of purification and the like, which are exemplarily defined by reference to certain alternative criteria, each of which is particularly preferred. Here, said levels may e.g. already be achieved by method step a). Likewise envisaged are respective levels resulting from the complete method, particularly since same may correspond to the former ones depending on the number of steps in the sample. In any case, also considered herein are any selections and combinations of said criteria - in particular because none of those criteria are considered mutually exclusive.

Accordingly, in preferred embodiments herein i-1) the small RNA molecules contained in the liquid phase obtained in step a) represent at least about 0.2%, 0,5%, 1%, 5%, 10%, preferably at least about 20%, preferably at least about 30%, more preferably at least about 40%, more preferably at least about 50%, more preferably at least about 60%, even more preferably at least about 70%, even more preferably at least about 80%, most preferably at least about 90% of the remaining solids of the said fungal and/or plant sample contained in the liquid phase; and/or i-2) the small RNA molecules contained in the sample resulting from the method represent at least about 0.2%, 0,5%, 1%, 5%, 10%, preferably at least about 20%, preferably at least about 30%, more preferably at least about 40%, more preferably at least about 50%, more preferably at least about 60%, even more preferably at least about 70%, even more preferably at least about 80%, even more preferably at least about 90%, most preferably at least about 95% of the remaining solids of the said fungal and/or plant sample, and/or ii-1) the small RNA molecules contained in the liquid phase obtained in step a) represent at least 0.2%, 0,5%, 1%, 5%, 80%, preferably at least 90%, more preferably at least 95%, even more preferably at least 98%, most preferably at most 99% of total nucleic acids contained in said liquid phase; and/or ii-2) the small RNA molecules contained in the sample resulting from the method represent at least 0.2%, 0,5%, 1%, 5%, 80%, preferably at least 90%, more preferably at least 95%, even more preferably at least 98%, most preferably at least 99% of total nucleic acids contained in the sample resulting from the method; and/or iii-1) the small RNA molecules contained in the liquid phase obtained in step a) represent at least 0.2%, 0,5%, 1%, 5%, 80%, preferably at least 90%, more preferably at least 95%, even more preferably at least 98%, most preferably at least 99% of total RNA contained in said liquid phase; and/or iii-2) the small RNA molecules contained in the sample resulting from the method represent at least 80%, preferably at least 90%, more preferably at least 95%, even more preferably at least 98%, most preferably at most 99% of total RNA contained in the sample resulting from the method, and/or iv-1) the DNA molecules contained in the liquid phase obtained in step a) represent at most 10%, more preferably at most 5%, even more preferably at most 2%, most preferably at most 1% of total nucleic acids contained in said liquid phase; and/or iv-2) the DNA molecules contained in the sample resulting from the method represent at most 10%, more preferably at most 5%, even more preferably at most 2%, most preferably at most 1% of total nucleic acids contained in the sample resulting from the method. Preferably herein, percentages indicate a weight percentage.

Accordingly, in preferred embodiments herein, the DNA molecules contained in the sample resulting from the method represent at most 10%, more preferably at most 5%, even more preferably at most 2%, most preferably at most 1% of total nucleic acids contained in the sample resulting from the method.

In certain related embodiments of a method of the invention, the RNA molecules consisting of 200 or more nucleotides contained in the sample resulting from the method represent at most 20%, preferably at most 10%, more preferably at most 5%, even more preferably at most 2%, most preferably at most 1% of total RNA contained in the sample resulting from the method.

In related embodiments of the present invention, the term "of total nucleic acids" is replaced by the term "of the total nucleic acid content". In related embodiments of the present invention, the term "of total nucleic acids" is replaced by the term "of the total content of DNA and RNA". In embodiments of the present invention, the term "of total RNA" is replaced by the term "of the total RNA content". In certain embodiments, any of the said contents refers to the content by weight. In certain alternative embodiments, any of the said contents refers the number of molecules.

Any of the methods of the invention may also be referred to herein as "new MRG extraction method(s)" or "new MRG methods" - as opposed to the related "previous MRG extraction method(s)" for isolating a fraction enriched of small RNA molecules involving the use of a solid support as e.g. disclosed in Applicant's previous application WO 2017/072285 (which is incorporated herein by reference in its entirety).

In further particular embodiments herein, the term(s) "method for purifying small RNA molecules from a sample" or "method for obtaining small RNA molecules from a sample" or "process for obtaining method small RNAs (sRNAs) containing extract" may be replaced by "method for enriching small RNA molecules in a sample". Exemplary embodiments of the latter include a method for enriching small RNA molecules in a sample, the method comprising the steps of: a) incubating a fungal and/or plant sample with a bicarbonate solution, to obtain a liquid phase containing small RNA molecules; b) adding an alcohol solution to the liquid phase obtained in step a) to precipitate small RNA molecules; and c) collecting, preferably by centrifuging, the precipitated small RNA molecules to obtain a sample enriched in small RNA molecules, with the proviso that the method does not employ a solid support for binding small RNA molecules.

A further step of adding an alcohol solution may be present in the method of in the invention, e.g. to the liquid phase obtained by a centrifugation carried out after step b).

Preferably in the methods of the invention, i) the method results in an enrichment of the small RNA molecules by a factor of at least 5, particularly at least 10, particularly at least 20, particularly at least 30, particularly at least 40, particularly at least 50, particularly at least 75, particularly at least 100, especially at least 500, in particular at least 1000, such as at least 5000, as compared to the starting sample, particularly if measured in weight percent (w/w); and/or ii) the percentage of small RNA molecules contained in the sample resulting from the method is increased by a factor of at least 5, particularly at least 10, particularly at least 20, particularly at least 30, particularly at least 40, particularly at least 50, particularly at least 75, particularly at least 100, especially at least 500, in particular at least 1000, such as at least 5000, as compared to the percentage in the starting sample, particularly if measured in weight percent (w/w); and/or iii) the small RNA molecules contained in the sample resulting from the method represent at least about 10%, preferably at least about 20%, preferably at least about 30%, more preferably at least about 40%, more preferably at least about 50%, more preferably at least about 60%, even more preferably at least about 70%, even more preferably at least about 80%, most preferably at least about 90% of total nucleic acids contained in the sample resulting from the method. In related embodiments, a method of the invention results in an enrichment of the small RNA molecules by a factor of at least 5, particularly of at least 10, particularly at least 20, particularly at least 30, particularly at least 40, particularly at least 50, particularly at least 75, particularly at least 100, especially at least 500, in particular at least 1000, such as at least 5000, as compared to the starting sample, particularly if measured in weight percent (w/w). In related embodiments of a method of the invention, the percentage of small RNA molecules contained in the sample resulting from the method is increased by a factor of at least 5, particularly of at least 10, particularly at least 20, particularly at least 30, particularly at least 40, particularly at least 50, particularly at least 75, particularly at least 100, especially at least 500, in particular at least 1000, 2000, 3000, such as at least 5000, as compared to the percentage in the starting sample, particularly if measured in weight percent (w/w).

In a third aspect, the present invention relates to methods of preparing a second product from a first product, wherein the product is preferably selected from the group consisting of a food, a food additive, a dietary supplement, a nutraceutical product, a particle, a microparticle, a nanoparticle, a pharmaceutical composition and a cosmetic composition, wherein the method comprises a method of the first or second aspect, and wherein the method further comprises adding small RNA molecules obtained by said method of any of the first and second aspect to said first product to obtain said second product, particularly wherein said second product additionally comprises a) lipids, more preferably at least one liposome, b) an exosome, c) a polymeric nanoparticle, more preferably a chitosan-based particle, and/or d) a β1,3-D-glucan particle.

Generally, in the third aspect, the present invention basically relates to methods of preparing a second product from a first product, wherein the method comprises a method of the invention, and wherein the method further comprises adding small RNA molecules obtained by said method of the invention to said first product to obtain said second product. Accordingly, in the third aspect, the present invention e.g. relates to methods of preparing a second product from a first product, wherein the method comprises a method of the first or second aspect, and wherein the method further comprises adding small RNA molecules obtained by said method of the first or second aspect to said first product to obtain said second product

Accordingly, in certain embodiments of the third aspect, the product is a food. In certain embodiments of this aspect, the product is a food additive. In certain embodiments of this aspect, the product is a dietary supplement. In certain embodiments of this aspect, the product is a nutraceutical product. In certain embodiments of this aspect the product is a dietary supplement.

In certain embodiments of the third aspect, the product is a nutraceutical product. Generally herein, further embodiments herein correspond to those involving a nutraceutical product, wherein the term "nutraceutical product" is replaced by the term "foodstuff".

In certain embodiments of the third aspect, the product is a particle. In certain embodiments of the third aspect, the product is a microparticle. In certain embodiments of the third aspect, the product is a nanoparticle. In certain embodiments of the third aspect, the product is a pharmaceutical composition. In certain embodiments of the third aspect, the product is a cosmetic composition.

In certain embodiments of the third aspect, said second product additionally comprises a) lipids, more preferably at least one liposome, b) an exosome, c) a polymeric nanoparticle, more preferably a chitosan-based particle, and/or d) a β1,3-D-glucan particle.

The product obtainable by the process according to the present invention may be comprised in any of the above disclosed product, preferably in a nutraceutical product, cosmetic product or pharmaceutical composition.

Generally, the sRNA molecules obtained by the present method may be used in the food or nutraceutical field. Generally, the sRNA molecules obtained by the present method may be used in the pharmaceutical field. Generally, the sRNA molecules obtained by the present method may be used in the field of cosmetics.

Generally, the sRNA molecules obtained by the present method may be used in the field of particles. Generally, the sRNA molecules obtained by the present method may be used in the field of microparticles. Generally, the sRNA molecules obtained by the present method may be used in the field of nanoparticles

According to the third aspect, the resulting second products herein, such as foods, nutraceutical products or foodstuffs, preferably contain an enlarged amount and/or concentration of plant or fungal sRNA molecules compared to the first product, such as foods nutraceutical products or foodstuffs, without addition of such food additive.

In a preferred embodiment, a second product, such as a nutraceutical product or foodstuff, contains an amount of plant or fungal sRNA or sRNA molecules which is at least 10% higher than the amount in the first product, such as nutraceutical product or foodstuff, without addition of such sRNA molecules, more preferably the amount is at least 20%, 50%, 100% or 200% higher than in the first product without such addition.

Foods, nutraceutical products or foodstuffs that include sRNA molecules in context with the invention for example comprise beverages, for instance sport drinks, fruiting juices, and alcoholic drinks as well as liquid preparation to be added to drinking water and liquid food. In another embodiment foods, nutraceutical products or foodstuffs comprise solid or semi-solid foods, e.g. baked goods, puddings, dairy products, confections, snack foods, or frozen confections or novelties (e.g., ice cream, milk shakes), prepared frozen meals, candy, snack products (e.g., chips), liquid food such as soups, spreads, sauces, salad dressings, prepared meat products, cheese, yogurt and any other fat or oil containing foods, and food ingredients (e.g., wheat flour). The term nutraceutical products or foodstuffs also includes functional foods and prepared nutraceutical products, the latter referring to any pre-packaged food approved for human consumption.

Foods, food additives, nutraceutical products and dietary supplements in context of the invention are preferably administered orally. Food additives, nutraceutical products and dietary supplements may be administered as single dose or multiple doses. Food additives, nutraceutical products and dietary supplements may be delivered in any suitable format, preferably for oral delivery. The ingredients of the dietary supplement of this invention are acceptable excipients and/or carriers for oral consumption. The carrier may be a liquid, gel, gelcap, capsule, powder, solid tablet (coated or non-coated), tea, or the like.

In other embodiments, a product such as a dietary supplement is prepared as a powder or liquid suitable for adding by the consumer to a food or beverage. For example, in some embodiments, the product such as the dietary supplement can be for administration to an individual in the form of a powder. Generally, sRNAs in context of the invention can be mixed into a beverage, or stirred into a semi-solid food, or otherwise be added to a product, such as a food. For instance, sRNAs in context of the invention can be enclosed in caps of food or beverage containers for release immediately before consumption.

The amount and dosage, respectively, of sRNA molecules of the invention in a product vary depending upon known factors, such as its mode and route of administration; the age, health and weight of the recipient; the nature and extent of the symptoms; the kind of concurrent treatment; the frequency of treatment; and the effect desired which can be determined by the expert in the field with normal trials. Typically, dosage amounts of the plant or fungal sRNA molecules may e.g. vary from 10-20 mg sRNA molecules per application to 1-2 mg sRNA molecules for daily use/application.

The pharmaceutical composition in context with the present invention can be administered in the form of a dosage unit, for example tablets or capsules, or a solution. The pharmaceutical compositions may be administered as single dose or multiple doses. Suitable routes of administration of the pharmaceutical composition in context with the invention include, for example, oral, intranasal and parenteral administration.

Suitable pharmaceutical carriers are e.g. described in Remington's Pharmaceutical Sciences, a standard reference text in this field. The pharmaceutical composition may further comprise conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like.

The compositions in context with the present invention may be in any galenic form that is suitable for administering to the animal body including the human body, more in particular in any form that is conventional for oral administration, e.g. in solid form, for example tablets, pills, granules, dragees, capsules, and effervescent formulations such as powders and tablets, or in liquid form, for instance in the form of solutions, emulsions or suspensions, for example as pastes and oily suspensions. The pastes may be filled into hard- or soft-shell capsules, whereby the capsules feature e.g. a matrix of (fish, swine, poultry, cow) gelatin, plant proteins or lignin sulfonate. Examples for other application forms are forms for transdermal, parenteral, topical, or injectable administration. The pharmaceutical compositions may be in the form of controlled (delayed) release formulations. Topical formulation may contain e.g. ointments, creams, gels, lotions, solutions. In the present invention the term "effective amount" shall mean an amount which achieves a desired effect or therapeutic effect as such effect is understood by those of ordinary skill in the art. For injection, including, without limitation, intravenous, intramuscular, and subcutaneous injection, the sRNA molecules in context with the invention may be formulated in aqueous solutions, preferably in physiologically compatible buffers such as physiological saline buffer or polar solvents including, without limitation, a pyrrolidone or dimethylsulfoxide.

Formulations for injection may be presented in unit dosage form, e.g. in ampoules or in multi-dose containers. Pharmaceutical compositions for parenteral administration include aqueous solutions of a water-soluble form, such as, without limitation, a salt of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances that increase the viscosity of the suspension, such as sodium carboxym ethyl cellulose, sorbitol, or dextran. Optionally, the suspension may also contain suitable stabilizers and/or agents that increase the solubility of the compounds to allow for the preparation of highly concentrated solutions. Alternatively, the active ingredient may be in powder form for constitution with a suitable vehicle, e.g., sterile, pyrogen- free water, before use.

For oral administration, the compounds can be formulated by combining the active compounds with pharmaceutically acceptable carriers well-known in the art. Such carriers enable the sRNA molecules of the invention and products in context with the invention, respectively, to be formulated as tablets, pills, lozenges, dragees, capsules, liquids, gels, syrups, pastes, slurries, solutions, suspensions, concentrated solutions, and suspensions for diluting in the drinking water of a patient, premixes for dilution in the feed of a patient, and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropyl-methylcellulose, sodium carboxymethylcellulose, and/or polyvinylpyrrolidone (PVP). If desired, disintegrating agents may be added, such as cross-linked polyvinyl pyrrolidone, agar, or alginic acid. A salt such as sodium alginate may also be used.

For administration by inhalation, the sRNA molecules of the present invention can conveniently be delivered in the form of an aerosol spray using a pressurized pack or a nebulizer and a suitable propellant. The sRNA molecules may also be formulated in rectal compositions such as suppositories or retention enemas, using, e.g., conventional suppository bases such as cocoa butter or other glycerides.

In addition to the formulations described previously, the sRNA molecules may also be formulated as depot preparations. Such long-acting formulations may be administered by implantation (for example, subcutaneously or intramuscularly) or by intramuscular injection. The sRNA molecules of the invention and products in context with the invention, respectively, may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

Determination of a therapeutically effective amount is well within the capability of those skilled in the art, especially in light of the disclosure herein.

The administration of such sRNA molecules may occur through complexing of the sequence with a suitable delivery system such as complexation with cationic liposomes or inclusion in nanovesicles. The delivery system can then be formulated for oral (including sub-lingual) administration as dietary supplement or directly integrated in food matrix or drinks for regular use. The daily doses may be in the range of 1-5 mg of active principle. The delivery system may be formulated for topical administration. The daily doses may be in the range of 1-5 mg of active principle. The delivery system may be formulated for intravenous administration with dosage over 10 mg of active principle.

The above defined sRNAs of 10 to 120 nucleotides, such as 15-60 bp of length extracted from plants or fungi may be functionalized to enhance their bioavailability and anti-inflammatory efficacy.

When dietary vegetables are consumed through the diet a small fraction of the microRNAs originally present in the food matrix passes through the GI tract and becomes available in the bloodstream (0.05%-0.5%). Such reduced bioavailability limits the anti-inflammatory efficacy towards human cells and organs of miRNAs and sRNAs naturally absorbed through the diet. Moreover, bioavailable dietary naked miRNAs or sRNAs may be subjected to degradation or structural modification within the bloodstream with ensuing loss of bioactivity.

In context with the present invention, it was further determined that e.g. complexation of sRNAs of 10 to 120 nucleotides, such as 15-60 bp sRNAs, extracted from plants or fungi with liposomes, exosomes or nanoparticles enables their effective uptake by dendritic cells of the human immune system. Moreover, complexation of such bioactive compounds will preserve integrity and reduce degradation in case of oral consumption during the mastication and passage through the GI tract, topic application during application on skin surface and permeation in the dermis, and intravenous application for movement within the bloodstream and transfer to recipient cells and organs. In case of oral consumption or topic application, complexation will also facilitate their transfer into the bloodstream. Complexation with cationic liposomes occurs naturally by physico-chemical interaction sRNAs are negatively charged in solution.

In a preferred embodiment the sRNAs as described above are provided within a delivery vehicle, optionally wherein the delivery vehicle is selected from liposomes, particularly cationic liposomes, liposome comprising lipids, or nanovesicles. Many methods are available for preparing liposomes, as described in, for example, Szoka et al, Ann. Rev. Biophys. Bioeng., 9:467 (1980) and U.S. Patents 4,235,871, 4,501,728, 4,837,028, and 5,019,369. In an embodiment, at least part of the lipids of the liposomes are selected from the group consisting of phospholipids, sterols, and sterol derivatives.

In a particular embodiment, the lipid of the liposomes comprises or constitutes a member selected from the group consisting of phosphatidylcholine (PC), phosphatidylethanolamine (PE), phosphatidylserine (PS), phosphatidylglycerol (PG), phosphatidylinositol (PI), phosphatidic acid (PA), DPG (bisphosphatidyl glycerol), PEOH (phosphatidyl alcohol), cholesterol, ergosterol and lanosterol. In a further embodiment, the liposome comprises phosphatidylcholines selected from the group consisting of 1,2-dioleoyl-phosphatidylcholine, 1,2-dipalmitoyl-phosphatidylcholine, 1,2-dimyristoyl-phosphatidylcholine, 1,2-distearoyl-phosphatidylcholine, 1-oleoyl-2-palmitoyl-phosphatidylcholine, 1-oleoyl-2-stearoyl-phosphatidylcholine, 1-palmitoyl-2-oleoyl-phosphatidylcholine, and 1-stearoyl-2-oleoyl-phosphatidylcholine.

In an embodiment the liposome comprises phosphatidylethanolamines selected from the group consisting of 1,2-dioleoyl-phosphatidylethanolamine, 1,2-dipalmitoyl-phosphatidylethanolamine, 1,2-dimyristoyl-phosphatidylethanolamine, 1,2-distearoyl-phosphatidylethanolamine, 1-oleoyl-2-palmitoyl-phosphatidylethanolamine, 1-oleoyl-2-stearoyl-phosphatidylethanolamine, 1-palmitoyl-2-oleoyl-phosphatidylethanolamine, 1-stearoyl-2-oleoyl-phosphatidylethanolamine and N-succinyl-dioleoyl-phosphatidylethanolamine; the phosphatidylserines are selected from the group consisting 1,2-dioleoyl-phosphatidylserine, 1,2-dipalmitoyl-phosphatidylserine, 1,2-dimyristoyl-phosphatidylserine, 1,2-distearoyl-phosphatidylserine, 1-oleoyl-2-palmitoyl-phosphatidylserine, 1-oleoyl-2-stearoyl-phosphatidylserine, 1-palmitoyl-2-oleoyl-phosphatidylserine and 1-stearoyl-2-oleoyl-phosphatidylserine; the phosphatidylglycerols are selected from the group consisting 1,2-dioleoyl-phosphatidylglycerol, 1,2-dipalmitoyl-phosphatidylglycerol, 1,2-dimyristoyl-phosphatidylglycerol, 1,2-distearoyl-phosphatidylglycerol, 1-oleoyl-2-palmitoyl-phosphatidylglycerol, 1-oleoyl-2-stearoyl-phosphatidylglycerol, 1-palmitoyl-2-oleoyl-phosphatidylglycerol and 1-stearoyl-2-oleoyl-phosphatidylglycerol; the phosphatidic acids are selected from the group consisting of di-palmitoyl-glycerophosphatidic acid, di-stearoyl-glycerophosphatidic acid, di-myrostoyl-glycerophosphatidic acid, di-oleoyl-glycerophosphatidic acid, palmitoyl-oleoyl-glycerophosphatidic acid.

In another embodiment of the liposome of the invention, the lipid comprises or constitutes phosphatidylglycerol (PG), phosphatidylethanolamine (PE), phosphatidylserine (PS), phosphatidylcholine (PC), phosphatidylinositol (PI), phosphatidic acid (PA), DPG (bisphosphatidyl glycerol), PEOH (phosphatidyl alcohol),cholesterol, phosphatidylcholines such as 1,2-dioleoyl-phosphatidylcho line, 1,2-dipalmitoyl-phosphatidylcho line, 1,2-dimyristoyl-phosphatidylcho line, 1,2-distearoyl-phosphatidylcholine, 1-oleoyl-2-palmitoyl-phosphatidylcholine, 1-oleoyl-2-stearoyl-phosphatidylcholine, 1-palmitoyl-2-oleoyl-phosphatidylcholine and 1-stearoyl-2-oleoyl-phosphatidylcholine; phosphatidylethanolamines such as 1,2-dioleoyl-phosphatidylethanolamine, 1,2-dipalmitoyl-phosphatidylethanolamine, 1,2-dimyristoyl-phosphatidylethanolamine, 1,2-distearoyl-phosphatidylethanolamine, 1-oleoyl-2-palmitoyl-phosphatidylethanolamine, 1-oleoyl-2-stearoyl-phosphatidylethanolamine, 1-palmitoyl-2-oleoyl-phosphatidylethanolamine, 1-stearoyl-2-oleoyl-phosphatidylethanolamine and N-succinyl-dioleoyl-phosphatidylethanolamine; phosphatidylserines such as 1,2-dioleoyl-phosphatidylserine, 1,2-dipalmitoyl-phosphatidylserine, 1,2-dimyristoyl-phosphatidylserine, 1,2-distearoyl-phosphatidylserine, 1-oleoyl-2-palmitoyl-phosphatidylserine, 1-oleoyl-2-stearoyl-phosphatidylserine, 1-palmitoyl-2-oleoyl-phosphatidylserine and 1-stearoyl-2-oleoyl-phosphatidylserine; phosphatidylglycerols such as 1,2-dioleoyl-phosphatidylglycerol, 1,2-dipalmitoyl-phosphatidylglycerol, 1 ,2-dimyristoyl-phosphatidylglycerol, 1,2-distearoyl-phosphatidylglycerol, 1-oleoyl-2-palmitoyl-phosphatidylglycerol, 1-oleoyl-2-stearoyl-phosphatidylglycerol, 1-palmitoyl-2-oleoyl-phosphatidylglycerol and 1-stearoyl-2-oleoyl-phosphatidylglycerol; 1,2-dioctadecanoyl-sn-glycero-3-ethylphosphocholine (Ethyl PC); pegylated lipids; pegylated phospoholipids such as phosphatidylethanolamine-N-[methoxy(polyethyleneglycol)-1000], phosphatidylethanolamine-N-[methoxy(polyethyleneglycol)-2000], phosphatidylethanolamine-N-[methoxy(poly ethyleneglycol)-3000], phosphatidylethanolamine-N-[methoxy(polyethyleneglycol)-5000]; pegylated ceramides such as N-octanoyl-sphingosine-1-{succinyl[methoxy(polyethyleneglycol)-1000]}, N-octanoyl-sphingosine-1-{succinyl[methoxy(polyethyleneglycol)2000]}, N-octanoyl-sphingosine-1-{succinyl[methoxy(polyethyleneglycol)3000]}, N-octanoyl-sphingosine-1-{succinyl[methoxy(polyethyleneglycol)5000]}; lyso-phosphatidylcholines, lysophosphatidylethanolamines, lyso-phosphatidylglycerols, lyso-phosphatidylserines, ceramides; sphingolipids; glycolipids such as ganglioside GMI; glucolipids; sulphatides; phosphatidic acid, such as di-palmitoyl-glycerophosphatidic acid; palmitic fatty acids; stearic fatty acids; arachidonic fatty acids; lauric fatty acids; myristic fatty acids; lauroleic fatty acids; physeteric fatty acids; myristoleic fatty acids; palmitoleic fatty acids; petroselinic fatty acids; oleic fatty acids; isolauric fatty acids; isomyristic fatty acids; isostearic fatty acids; sterol and sterol derivatives such as cholesterol, cholesterol hemisuccinate, cholesterol sulphate, and cholesteryl-(4-trimethylammonio)-butanoate, ergosterol, lanosterol; polyoxyethylene fatty acids esters and polyoxyethylene fatty acids alcohols; polyoxyethylene fatty acids alcohol ethers; polyoxyethylated sorbitan fatty acid esters, glycerol polyethylene glycol oxy-stearate; glycerol polyethylene glycol ricinoleate; ethoxylated soybean sterols; ethoxylated castor oil; polyoxyethylene polyoxypropylene fatty acid polymers; polyoxyethylene fatty acid stearates; di-oleoyl-sn-glycerol; dipalmitoyl-succinylglycerol; 1,3-dipalmitoyl-2-succinylglycerol; 1-alkyl-2-acyl-phosphatidylcholines such as 1-hexadecyl-2-palmitoyl-phosphatidylcholine; 1-alkyl-2-acyl-phosphatidylethanolamines such as 1-hexadecyl-2-palmitoyl-phosphatidylethanolamine; 1-alkyl-2-acyl-phosphatidylserines such as 1-hexadecyl-2-palmitoyl-phosphatidylserine; 1-alkyl-2-acyl-phosphatidylglycerols such as 1-hexadecyl-2-palmitoyl-phosphatidylglycerol; 1-alkyl-2-alkyl-phosphatidylcholines such as 1-hexadecyl-2-hexadecyl-phosphatidylcholine; 1-alkyl-2-alkyl-phosphatidylethanolamines such as 1-hexadecyl-2-hexadecyl-phosphatidylethanolamine; 1-alkyl-2-alkyl-phosphatidylserines such as 1-hexadecyl-2-hexadecyl-phosphatidylserine; 1-alkyl-2-alkyl-phosphatidylglycerols such as 1-hexadecyl-2-hexadecyl-phosphatidylglycerol; and N-Succinyl-dioctadecylamine; palmitoylhomocysteine.

An embodiment relates to a liposome, wherein at least part of the lipids is a cationic lipid. An embodiment relates to a liposome, wherein the cationic lipids are selected from the group consisting of stearylamine (SA), lauryltrimethylammonium bromide; cetyltrimethylammonium bromide, myristyl trimethylammonium bromide, dimethyldioctadecylammonium bromide (DDAB), 3β-[N—(N',N'-dimethylaminoethane)-carbamoyl]cholesterol (DC-Cholesterol), 1,2-ditetradecanoyl-3-trimethylammonium-propane (DMTAP), 1,2-dioctadecanoyl-3-trimethylammonium-propane (DOTAP) and DOTAP derivatives such as 1,2-di-(9Z-octadecenoyl)-3-trimethylammonium-propane and 1,2-dihexadecanoyl-3-trimethylammonium-propane, 1,2-di-(9Z-octadecenoyl)-3-dimethylammonium-propane (DODAP) and DODAP derivatives such as 1,2-ditetradecanoyl-3-dimethylammonium-propane, 1,2-dihexadecanoyl-3-dimethylammonium-propane, and 1,2-dioctadecanoyl-3-dimethylammonium-propane, 1,2-di-O-octadecenyl-3-trimethylammoniumpropane (DOTMA), 1,2-dioleoyl-c-(4'-trimethylammonium)-butanoyl-sn-glycerol (DOTB), dioctadecylamide-glycylspermine, SAINT-2, polycationic lipid 2,3-dioleyloxy-N-[2(spermine-carboxamido)ethyl]-N,N-dimethyl-1-propanaminiumtrifluoroacetate (DOSPA), and GL67TM.

According to particular embodiments of the liposome in context with the invention, the cationic lipids are selected from the group consisting of stearylamine (SA), 1,2-dioctadecanoyl-3-trimethylammonium-propane (DOTAP) and 1,2-di-(9Z-octadecenoyl)-3-dimethylammonium-propane (DODAP), preferably 1,2-dioctadecanoyl-3-trimethylammonium-propane (DOTAP). Preferred cationic lipids are DOTAP and DOTAP derivatives. Additional examples of cationic lipids and lipid components may be found in or made according to U.S. Pat. No. 4,804,539. According to particular embodiments of the liposome in context with the invention, at least part of the lipids is a cationic lipopeptide selected from the group consisting of a lipid polyarginine conjugate, a lipid TAT conjugate, a lipid polylysine conjugate, or a cationic liposaccharide or lipopolysaccharide such as a lipid chitosan conjugate. In a preferred embodiment, the lipids are capable of forming a liposome. In particular, cationic lipids are suitable for this purpose. Cationic lipids preferably include DOTAP, DOPE, DC-Chol/DOPE, DOTMA, and DOTMA/DOPE. Cationic Lipids are well known to the person skilled in the art. Cationic lipids carry a net positive charge at about physiological pH. Suitable cationic lipids include, but are not limited to, N,N-dioleyl-N,N-dimethylammonium chloride ("DODAC"); N-(2,3-dioleyloxyl)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP") ; 1,2-Dioleyloxy-3-trimethylaminopropane chloride salt ("DOTAP.C1"); 3P-(N-(N',N'-dimethylaminoethane)-carbamoyl)cholesterol ("DC-Choi"), N-(1-(2,3-dioleyloxyl)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N, N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, several commercial preparations of cationic lipids can be used, such as, e.g., LIPOFECTIN (including DOTMA and DOPE, available from GIBCO/BRL), and LIPOFECTAMINE (comprising DOSPA and DOPE, available from GIBCO/BRL). In particular embodiments, a cationic lipid is an amino lipid. Additional cationic lipids include l,2-dilinoleyloxy-3-dimethylaminopropane (DLinDMA) 1,2-Dilinoleoyl-3-dimethylaminopropane (DLinDAP), 1-Linoleoyl-2-linoeyloxy-3-dimethylaminopropane (DLin-2-DMAP), 1,2-Dilinoleylcarbamoyloxy-3-dimethylaminopropane (DLin-C-DAP), 1,2-Dilinoleylthio-3-dimethylaminopropane (DLin-S-DMA), 2,2-Dilinoleyl-4-dimethylamino methyl-[1 ,3]-dioxolane (DLin-K-DMA).

An embodiment of the invention involves a liposome or a vesicle, with a particle size of between 0.1 and 100 µm. The liposome of vesicle may comprise the small RNA molecules obtained by a method of the invention.

In certain embodiments, the ratio of total lipid to sRNA is from 5 to 35 (i.e. from 5 to 1 to 35 to 1, lipid weight to sRNA weight). In certain embodiments, the ratio of total lipid to sRNA is from 5 to 15 (i.e. from 5 to 1 to 15 to 1, lipid weight to sRNA weight).

In a yet further preferred embodiment, exosomes may be used. Such exosomes and their preparation are described e.g. in Montecalvo et al. (2012, Blood, 119: 756-766, and Stoorvogel, 2012, Blood, 119: 646-648). For example, exosomes loaded with plant or fungal sRNA or sRNA extract(s) or compositions comprising 2 or more plant or fungal sRNA, or sRNA extract(s) may be used. For example, exosomes loaded with plant miRNA may be used.

Polymeric nanoparticles formed by self-assembly of polycations with siRNA can be used for extracellular delivery, cellular uptake, and intracellular trafficking as a strategy to improve the therapeutic potential of siRNA. Polycationic polymer-based nanoparticle (or polyplex) systems used for site-specific delivery, cellular uptake, and intracellular trafficking of siRNA. Such nanoparticles and in particular chitosan-based particles and their preparations are described e.g. in Ostergaard et al., Therapeutic Applications of RNAi: Methods and Protocols, Humana Press 2009. For example, nanoparticles loaded with plant or fungal sRNA or sRNA extract(s) or compositions comprising 2 or more plant or fungal sRNA or sRNA extract(s) may be used.

Another delivery strategy is via β 1,3-D-glucan particles (GP), hollow and porous microspheres derived from Saccharomyces cerevisiae (Baker's yeast) that provide an efficient system for encapsulation, protection, and oral or systemic macrophage-targeted delivery of macromolecules, such as DNA, siRNA and proteins using either a polyplex or layer-by- layer (LbL) synthesis methods. Such particles and their preparations are described e.g. in Soto and Ostroff, Nanomaterials for Biomedicine, 3, 57-79 2012. In a particular embodiment, plant or fungal sRNA is encapsulated in 2-4 µm hollow β 1,3-D-glucan particles. For example, glucan particles loaded with plant or fungal sRNA molecules of the invention may be used.

In a fourth aspect, the present invention relates to the use of bicarbonate solution in a method of the invention, i.e. in particular to the use of bicarbonate solution in a method of the first or second aspect as defined herein above.

In detail, said fourth aspect relates to the use of a bicarbonate solution in a method I) for purifying small RNA molecules from a fungal and/or plant sample comprising the step of: a) incubating said sample with said bicarbonate solution to obtain a liquid phase containing purified small RNA molecules; and/or II) for obtaining small RNA molecules from a fungal and/or plant sample comprising the step of: a) incubating said sample with said bicarbonate solution to obtain a liquid phase containing small RNA molecules thus obtaining said small RNA molecules; and/or III) for increasing the ratio of small RNA molecules to total RNA in a fungal and/or plant sample comprising the step of: a) incubating said sample with said bicarbonate solution to obtain a liquid phase having an increased ratio of small RNA molecules to total RNA; and/or IV) for increasing the ratio of small RNA molecules to total nucleic acids in a fungal and/or plant sample comprising the step of: a) incubating said sample with said bicarbonate solution to obtain a liquid phase having an increased ratio of small RNA molecules to total nucleic acids; particularly with the proviso that the said method(s) does/do not employ a solid support for binding small RNA molecules, especially wherein said methods is/are further defined as in the first and/or second aspects herein.

Accordingly, in certain embodiments, said fourth aspect relates to the use of a bicarbonate solution in a method for purifying small RNA molecules from a fungal and/or plant sample comprising the step of: a) incubating said sample with said bicarbonate solution to obtain a liquid phase containing purified small RNA molecules, particularly wherein said method is further defined in accordance with any other aspect herein, especially in accordance with the first and/or second aspect.

Accordingly, in certain embodiments, said fourth aspect relates to the use of a bicarbonate solution in a method for obtaining small RNA molecules from a fungal and/or plant sample comprising the step of: a) incubating said sample with said bicarbonate solution to obtain a liquid phase containing small RNA molecules thus obtaining said small RNA molecules, particularly wherein said method is further defined in accordance with any other aspect herein.

Accordingly, in certain embodiments, said fourth aspect relates to the use of a bicarbonate solution in a method for increasing the ratio of small RNA molecules to total RNA in a fungal and/or plant sample comprising the step of: a) incubating said sample with said bicarbonate solution to obtain a liquid phase having an increased ratio of small RNA molecules to total RNA, particularly wherein said method is further defined in accordance with any other aspect herein.

Accordingly, in certain embodiments, said fourth aspect relates to the use of a bicarbonate solution in a method for increasing the ratio of small RNA molecules to total nucleic acids in a fungal and/or plant sample comprising the step of: a) incubating said sample with said bicarbonate solution to obtain a liquid phase having an increased ratio of small RNA molecules to total nucleic acids; particularly with the proviso that the said method(s) does/do not employ a solid support for binding small RNA molecules, particularly wherein said method is further defined in accordance with any other aspect herein.

In particular embodiments of the fourth aspect, there is provided the use of a bicarbonate solution in a method for purifying small RNA molecules from a sample comprising the steps of: a) incubating a fungal and/or plant sample with said bicarbonate solution to obtain a liquid phase containing small RNA molecules; b) adding an alcohol solution to the liquid phase obtained in step a) to precipitate small RNA molecules; and c) centrifuging the precipitated small RNA molecules to obtain a sample containing purified small RNA molecules, with the proviso that the said method does not employ a solid support for binding small RNA molecules, particularly wherein said method is further defined as described hereinabove.

In particular certain embodiments of the fourth aspect, there is provided the use of a bicarbonate solution in a method for obtaining, preferably for selectively obtaining, small RNA molecules from a fungal and/or plant sample comprising the steps of: a) incubating said sample with said bicarbonate solution to obtain a liquid phase containing small RNA molecules; b) adding an alcohol solution to the liquid phase obtained in step a) to precipitate small RNA molecules; and c) centrifuging the precipitated small RNA molecules to obtain the small RNA molecules; with the proviso that the said method does not employ a solid support for binding small RNA molecules, particularly wherein said method is further defined as described hereinabove.

Preferably herein, the use of the fourth aspect is i) for increasing the ratio of small RNA molecules to total RNA, and/or ii) for increasing the ratio of small RNA molecules to total nucleic acids, and/or iii) for isolating small RNA molecules from a sample; particularly wherein the said ratio indicates a weight ratio.

In certain embodiments, the use of the fourth aspect is for improving the yield of obtained small RNA molecules. In certain embodiments, said use is for purifying the small RNA molecules. In certain embodiments, said use is for obtaining, preferably selectively obtaining, the small RNA molecules. In certain embodiments, said use is for increasing the percentage of small RNA molecules contained in the sample. In certain embodiments, said use is for improving the purity of obtained small RNA molecules. In certain embodiments, said use is for separating the small RNA molecules from other RNA molecules in the sample. In certain embodiments, said use is for separating the small RNA molecules from DNA molecules in the sample. In certain embodiments, said use is for avoiding the use of toxic reagents. In certain embodiments, said use is for achieving, especially for improving, the release, particularly the selective release, of small RNA molecules from the sample.

In an even further aspect, the present invention concerns kits for isolating small RNA molecules (preferably small RNA molecules as defined above), such as miRNA and/or siRNA from a fungal or plant sample, particularly a cell or tissue sample.

Here, any of the compositions discussed above can be included with any other composition discussed above for inclusion in a kit. In some embodiments of the latter aspect, the kit comprises: i) a bicarbonate solution (preferably as defined above), ii) an alcohol solution (preferably as defined above), and optionally iii) one or more wash solution(s), and optionally iv) an aqueous solution, preferably an RNase-free aqueous solution, and optionally v) one or more RNase inhibitors (preferably as defined above). Preferably, the kit in accordance with the invention is characterized in that it does not contain a solid support (preferably as defined above).

The components of the kits may be packaged either in aqueous media or in lyophilized form. The container means of the kits will generally include at least one vial, test tube, flask, bottle, syringe, or other container means, into which a component may be placed, and preferably, suitably aliquoted. Where there is more than one component in the kit (they may be packaged together), the kit will generally also contain a second, third or other additional container into which the additional components may be separately placed. However, various combinations of components may be comprised in a vial. The kits of the present invention also will typically include a means for containing the RNA, and any other reagent containers in close confinement for commercial sale. Such containers may include injection or blow-molded plastic containers into which the desired vials are retained. When the components of the kit are provided in one and/or more liquid solutions, the liquid solution is an aqueous solution, with a sterile aqueous solution being particularly preferred.

However, the components of the kit may be provided as dried powder (s). When reagents and/or components are provided as a dry powder, the powder can be reconstituted by the addition of a suitable solvent. It is envisioned that the solvent may also be provided in another container means. The container means will generally include at least one vial, test tube, flask, bottle, syringe and/or other container means, into which the nucleic acid formulations are placed, preferably, suitably allocated. The kits may also comprise a second container means for containing a sterile, pharmaceutically acceptable buffer and/or other diluent. Such kits may also include components that preserve or maintain the RNA or that protect against its degradation. Such components may be RNase-free or protect against RNases. Such kits generally will comprise, in suitable means, distinct containers for each individual reagent or solution.

With respect to centrifugation steps employed in the present invention, the person skilled in the art will readily be able to select a suitable centrifugation mode - and particularly also in view of the data described in the Examples herein, which modes correspond to certain preferred embodiments herein. In general preferred embodiments herein, centrifugation is carried out at least at 2.500g, and preferably at 2.500g-10.000g.

In the context of the present invention the term "comprising" includes the terms "comprising", "consisting of" and "consisting essentially of".

Generally, herein, further embodiments of a given aspect correspond to embodiments of any other aspect herein.

Generally, herein, further embodiments may be derived from the features (i.e. from individual features or from any combination of features) disclosed in the present Examples.

### Examples

The following examples are meant to further illustrate, but not limit, the invention. The examples comprise technical features, and it will be appreciated that the invention also relates to any combinations of the technical features presented in this exemplifying section.

### Example 1

This example provides data for certain methods of the present invention, which may also be-called "new MRG extraction method(s)", which e.g. show that the successful and advantageous isolation / purification of small RNAs from plant and/or fungal samples is possible without the use of a solid support for binding sRNA molecules. Such an exemplary method is described in the following in direct comparison with various related methods that do use a solid support for binding sRNA molecules.

### Method 1 (not using a solid support)

1 Incubate intact or coarsely chopped plant/fungal sample in bicarbonate solution for 2-6h at RT.
   (The bicarbonate solution included 10, 30 or 50 of mM NaHCO₃ and 0.15, 0.2 or 0.25 M of NaCI).
2 Add other bicarbonate solution and raise the temperature at 60-65°C for few hours.
3 Filter to obtain liquid phase and discard solid phase
4 Allow liquid phase to cool off
5 Add cold isopropanol
6 Centrifuge ≥5,000 rcf, ≥10 min
7 Remove and discard liquid phase
8 Wash pellet with ethanol, centrifuge and remove liquid phase
9 Dry pellet
10 Add distilled water to resuspend pellet
11 Centrifuge
12 Collect liquid phase containing sRNA fraction

Moreover, to compare this method to correspondingly adapted protocols which use solid supports, further experiments were performed. The methods which use solid support were adapted to the above method 1 to facilitate the comparison, i.e. the initial step(s) of the methods were performed in line with those of method 1 above.

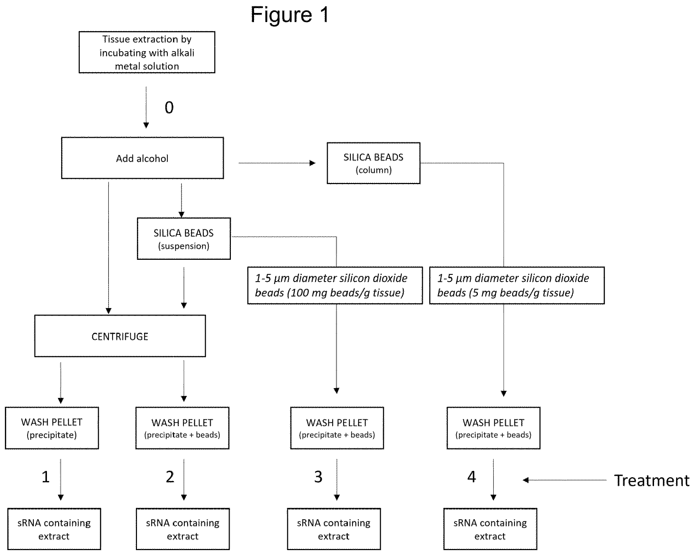

A summary scheme including method 1 and comparative methods 2 to 4 is shown in Figure 1.

The sRNA content of the final extract was determined using the Agilent RNA 6000 Nano Kit (through the Bioanalyzer instrument /Agilent Technologies) and data are expressed as mg sRNA obtained from 1 kg of tissue.

In detail, comparative methods 2, 3 and 4 were performed according to the following protocols:

### Method 2 (using a solid support)

1 1-5 as above for Method 1.
2 Add 1-5 µm diameter silicon dioxide beads - for beads preparation: Amorphous silica Sigma 274739 (50-70 mesh) is resuspended with 10% HCI,and allowed to settle for 24 h. The supernatant is aspirated and discarded. The pellet is resuspended with 6 ml of 0.1 M HCI and then aliquoted and stored at 4 °C) (100 mg/g tissue.
3 Incubate at T ≤ 4°C for at least 30 min with agitation
4 Steps 6-11 as above
5 Collect the liquid phase containing sRNA fraction

### Method 3 (using a solid support)

1 Steps 1-5 as above for Method 1
2 Add 1-5 µm diameter silicon dioxide beads (preparation of beads as above)
3 Incubate at T ≤ 4°C for at least 30 min with agitation
4 Let the solids decant and remove and discard liquid phase
5 Dry solids with air flow
6 Wash solids with ethanol (at least 2:1 v/v depending on solids size)
7 Let the solids decant and remove and discard liquid phase
8 Dry solids with air flow
9 Add distilled water
10 Let solids decant
11 Collect liquid phase containing sRNA fraction

In addition, variants of Method 3 were performed, which differ in the type of addition of silica beads.

### Method 4 (using a solid support)

1 Steps 1-5 as above for Method 1
2 Pour liquid phase into a column containing 1-5 µm diameter silicon dioxide beads (preparation as above)
3 Discard eluate.
4 Wash column with 70% ethanol (at least 2:1 v/v)
5 Discard eluate
6 Wash column with distilled water (at least 2:1 v/v)
7 Collect eluate containing sRNA fraction

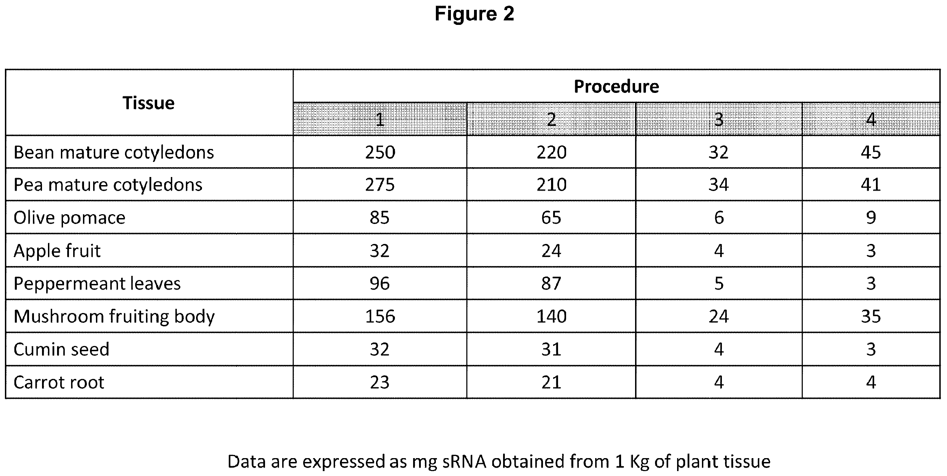

The methods were performed on a multitude of plants and fungal samples and the results are summarized in Fig 2.

The results demonstrate that the methods of the invention may advantageously be used for large scale preparation of small RNAs, such as from 1 kg of plant tissue. In addition, the results show that the yields of small RNAs obtained with Method 1 are (unexpectedly) higher with respect to the ones obtained with the other extraction methods (using a solid support - Methods 2-4) for all the vegetal materials tested.

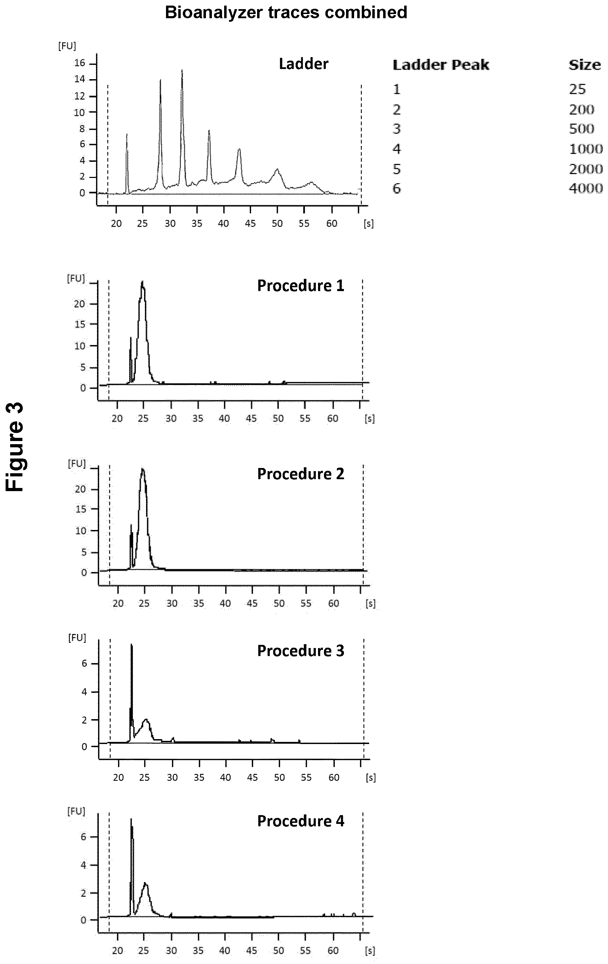

The results have been also confirmed by analyzing the RNA profile of the extracts by using Bioanalyzer instrument (Agilent Technologies) as shown in Fig.3.

Accordingly, with the methods of the invention, it is possible to obtain small RNA molecules without the use of a solid support.

### Example 2

This example provides further data for particular methods of the present invention, i.e. socalled "new MRG extraction method(s)", which show that the purification of small RNAs from plant and/or fungal samples is possible by essentially using a step of incubating a sample in bicarbonate solution to obtain a liquid phase containing (purified) sRNA molecules, i.e. without using subsequent steps involving alcohol precipitation or the like. Figure 5 consists of two parts and provides comparative data with respect to a non-limiting exemplary method of the present invention only including bicarbonate extraction (cf. Figure 5a and 5c) and a non-limiting exemplary method that additionally includes further steps such as alcohol precipitation (cf. Figure 5b).

The RNA profiles were obtained through a Bioanalyzer instrument (Agilent Technologies) using the RNA 6000 Nano Kit (figure 5a) or the small RNA Kit (figure 5c) are shown. As indicated, the small RNA fraction (20-200nt) represents 98% of the total RNA content in the extract. In the total RNA profile (Fig. 5a) the small RNA component represents the 98% (compared to the 10% of starting tissue (see Fig. 4)). Moreover, the distribution of the length classes of sRNAs (Fig. 5c) shows 92% of form ranging from 10 and 40 nt.

Figure 4 shows the profiles of total RNA isolated by using Spectrum^{™} Plant Total RNA (Sigma-Aldrich) according to manufacturer's instructions. Profiles obtained through a Bioanalyzer instrument (Agilent Technologies) using the RNA 6000 Nano Kit (Fig4a) or the small RNA Kit (Fig.4b) are shown, along with the percentages of RNA fractions of different length (20-200nt; >200nt). Small RNA represents about the 10% of the total (Fig. 4a). The distribution of the length classes of sRNAs (Fig. 4b) shows the almost total absence of RNA forms below 40nt (1% of the total).

Accordingly, the results above show that it is unexpectedly possible to purify (or obtain, respectively) small RNA molecules using methods of the invention just using a step of incubating a sample with a bicarbonate solution to obtain a liquid phase containing (purified) small RNA molecules.

Moreover, the results above further confirm that, with the methods of the invention, it is possible to easily obtain small RNA molecules without using the (toxic) chemicals that are employed by the methods of the prior art.

Besides, the results above show that "step 0" depicted in Figure 1 may be employed in the present methods for purifying / obtaining sRNA molecules of the present invention also in the absence of downstream method steps, such as in the absence of alcohol precipitation steps.

## Claims

1. A method for obtaining a small RNAs (sRNAs) containing extract from fungi and/or plant or part thereof, said extract being **characterized by** a fraction enriched of sRNA molecules said sRNA molecules having less than 200 nucleotides or base pairs (bp), wherein said process comprises:
a) at least one step of treating said fungi or plant or part thereof with a bicarbonate solution, to obtain a liquid phase comprising or consisting of a small RNA containing extract, providing that said method does not employ a solid support for binding small RNA molecules.

2. The method according to claim 1 further comprising:
b') at least one step of collecting the liquid phase of step a) and concentrating it to obtain a concentrated small RNAs containing extract; and/or
b) at least one step of collecting the liquid phase of step a) or the concentrated small RNAs containing extract of step b') and adding an alcohol solution to said liquid phase of step a) or to said concentrated small RNAs containing extract of step b') to allow precipitation of the small RNAs containing extract;
and optionally the following step:
c) collecting the concentrated small RNAs containing extract of step b') or the precipitated sRNAs containing extract of step b).

3. The method according to claim 1 or 2, wherein the sRNA molecules are **characterized by** less than 100 nucleotides, more preferably less than 30 nucleotides or bp; still more preferably said sRNAs having 19-24 nucleotides or bp, still more preferably 21-24 nucleotides or bp.

4. The method according to anyone of claims 1-3, wherein the sRNA molecules represent up to 300 mg/Kg of plant or fungi processed, preferably up to 250 mg/Kg of plant or fungi processed, more preferably up to 200 mg/Kg of plant or fungi processed, still more preferably up to 100 mg/Kg of plant or fungi processed.

5. The method according to anyone of claims 1-4, wherein said bicarbonate solution is selected from the group consisting of sodium bicarbonate, potassium bicarbonate, and mixtures thereof.

6. The method according to anyone of claims 1-5, wherein said bicarbonate solution is used at a concentration ranging between 5 and 100 mM, preferably between 5 and 50 mM, more preferably between 5 and 30 mM, still more preferably between 5 and 10 mM or between 10 and 30 mM.

7. The method according to anyone of claims 1-6, wherein said bicarbonate solution further comprises a salt, preferably NaCl, wherein said salt, preferably NaCl, is present in a concentration preferably ranging between 100 and 500 mM.

8. The method according to anyone of claims 1-7, wherein said bicarbonate solution shows a pH which ranges between 7 and 9.5.

9. The method according to anyone of claims 1-8, wherein the ratio between said fungi/plant and the bicarbonate solution ranges between 1:1 and 1:4.

10. The method according to anyone of claims 1-9, wherein step a) is performed at a temperature ranging between 50 to 100°C, preferably from 50 to 80°C, more preferably from 50 to 70°C, even more preferably from 60 to 70°C.

11. The method according to anyone of claims 1-10, wherein step a) is performed for up to 20 hours, preferably up to 16 hours, more preferably up to 10 hours, still more preferably up to 6 or less.

12. The method according to anyone of claims 1-11, wherein the liquid phase is collected by using any mean known in the art for this purpose, preferably by filtration, preferably with filters having pores with size of 10-50 µm.

13. The method according to anyone of claims 1-12, wherein said alcohol is selected from isopropanol and/or ethanol and it is preferably used at a final concentration selected from: 98%, 97%, 96%, 95%, 90%, 85%, 80%, 75%, 70%, 65%, 60%, 55%, 50% v/v.

14. The method according to anyone of claims 1-13, wherein the ratio liquid part: alcohol is selected from: 0.5:1, 1:1, 1,5:1, 2:1, 2,5:1, 3:1, 3,5:1, 4:1, 4,5:1.

15. A product obtainable by the method according to any one of the previous claims.
